# EUROPEAN PATENT SPECIFICATION

(11) **EP 3 013 944 B1**
(45) Date of publication and mention of the grant of the patent: **18.03.2020**
(21) Application number: 14818738.8
(22) Date of filing: 24.06.2014
(51) Int. Cl.: C12N 5/077, C12N 5/0775, A61K 35/28, A61P 17/02, A61P 29/00, A61P 37/00

(54) **COMPOSITION OF MESENCHYMAL STEM CELLS**
ZUSAMMENSETZUNG AUS MESENCHYMALEN STAMMZELLEN
COMPOSITION DE CELLULES SOUCHES MÉSENCHYMATEUSES

(30) Priority: 24.06.2013 US 201361838827 P
(43) Date of publication of application: 04.05.2016
(73) Proprietor: University of Southern California, Los Angeles, CA 90015 (US)
(72) Inventor: SHI, Songtao, Thousand Oaks, CA 91362 (US); XU, Xingtian, South Pasadena, CA 91030 (US); CHEN, Chider, Philadelphia, PA 19104 (US); LE, Anh D., La Mirada, CA 90638 (US)
(74) Representative: Müller-Boré & Partner Patentanwälte PartG mbB
(86) International application number: PCT/US2014/043918
(87) International publication number: WO 2014/210037

(56) References cited:
- WO-A1-2013/076726
- US-A1- 2012 128 636
- KEREN MARYNKA-KALMANI ET AL: "The Lamina Propria of Adult Human Oral Mucosa Harbors a Novel Stem Cell Population", STEM CELLS, vol. 28, no. 5, 1 May 2010 (2010-05-01), pages N/A-N/A, XP055044659, ISSN: 1066-5099, DOI: 10.1002/stem.425
- QUN-ZHOU ZHANG ET AL: "Human Gingiva-Derived Mesenchymal Stem Cells Elicit Polarization of M2 Macrophages and Enhance Cutaneous Wound Healing", STEM CELLS., vol. 28, no. 10, 1 October 2010 (2010-10-01), pages 1856-1868, XP55317074, US ISSN: 1066-5099, DOI: 10.1002/stem.503
- TOMAR ET AL.: 'Human gingiva-derived mesenchymal stem cells are superior to bone marrow- derived mesenchymal stem cells for cell therapy in regenerative medicine.' BIOCHEM BIOPHYS RES COMMUN. vol. 393, 12 March 2010, pages 377 - 383, XP026960849

## Description

This disclosure relates in general to mesenchymal stem cell therapy. This disclosure further relates to the isolation and application of gingiva derived mesenchymal stem cells. More particularly, this disclosure relates to the isolation and applications of the neural crest derived gingiva mesenchymal stem cells and mesoderm derived gingiva mesenchymal stem cells.

Mesenchymal stem cells (MSCs) possess multipotent differentiation potential and capability to regulate immune response. For example, see Uccelli et al. "Mesenchymal stem cells in health and disease" (2008) Nat. Rev. Immunol. 8:726-736; Nauta et al. "Immunomodulatory properties of mesenchymal stromal cells" (2007) Blood 110:3499-3506; and Aggarwal et al. "Human mesenchymal stem cells modulate allogeneic immune cell responses" (2005) Blood 105:1815-1822. Cell types that MSCs have been shown to differentiate into in vitro or in vivo include osteoblasts, chondrocytes, myocytes, adipocytes, endotheliums, and beta-pancreatic islets cells.

Mesenchymal stem cells are characterized morphologically by a small cell body with a few cell processes that are long and thin. The cell body contains a large, round nucleus with a prominent nucleolus which is surrounded by finely dispersed chromatin particles, giving the nucleus a clear appearance. The remainder of the cell body contains a small amount of Golgi apparatus, rough endoplasmic reticulum, mitochondria, and polyribosomes. The cells, which are long and thin, are widely dispersed and the adjacent extracellular matrix is populated by a few reticular fibrils but is devoid of the other types of collagen fibrils.

MSCs have a large capacity for self-renewal while maintaining their multipotency. The standard test to confirm multipotency is differentiation of the cells into osteoblasts, adipocytes, and chondrocytes as well as myocytes and possibly neuron-like cells. However, the degree to which the culture will differentiate varies among individuals and how differentiation is induced, e.g. chemical vs. mechanical. The capacity of cells to proliferate and differentiate is known to decrease with the age of the donor, as well as the time in culture.

It was disclosed that human MSCs may avoid allorecognition, interfere with dendritic cell and T-cell function and generate a local immunosuppressive microenvironment by secreting cytokines. It was also disclosed that the immunomodulatory function of human MSCs may be enhanced when the cells are exposed to an inflammatory environment characterized by the presence of elevated local interferon-gamma levels.

Thus, MSCs may be used in tissue regeneration and immune therapies. For example, see Le Blanc et al. "Mesenchymal stem cells for treatment of steroid-resistant, severe, acute graft-versus-host disease: a phase II study" (2008) Lancet 371:1579-1586; Sun et al. "Mesenchymal stem cell transplantation reverses multiorgan dysfunction in systemic lupus erythematosus mice and humans" (2009) Stem Cells 27:1421-1432; and Akiyama et al. "Mesenchymal-stem-cell-induced immunoregulation involves FAS-ligand-/FAS-mediated T cell apoptosis" (2012) Cell Stem Cell 10:544-555.

The orofacial region contains a variety of distinctive MSC populations, including dental pulp stem cells, stem cells from deciduous dental pulp, periodontal ligament stem cells, apical papilla stem cells, and dental follicle stem cells. For example, see Gronthos et al. "Postnatal human dental pulp stem cells (DPSCs) in vitro and in vivo" (2000) Proc. Natl. Acad. Sci. USA 97:13625-13630; Miura et al. "SHED: stem cells from human exfoliated deciduous teeth" (2003) Proc. Natl. Acad. Sci. USA 100:5807-5812; Yamaza et al. "Immunomodulatory properties of stem cells from human exfoliated deciduous teeth" (2011) Stem Cell Res. Ther. 1:5-14; Seo et al. "Investigation of multipotent postnatal stem cells from human periodontal ligament" (2004) Lancet 364:149-155; and Morsczeck et al. "Isolation of precursor cells (PCs) from human dental follicle of wisdom teeth" (2005) Matrix Biol. 24:155-65.

Recently, gingiva/mucosa-derived mesenchymal stem cells (GMSCs) were isolated and characterized as having multi-lineage differentiation capacity and immunomodulatory properties. For example, see Zhang et al. "Mesenchymal stem cells derived from human gingiva are capable of immunomodulatory functions and ameliorate inflammation-related tissue destruction in experimental colitis" (2009) J. Immunol. 183:7787-7798; Zhang et al. "Human gingiva-derived mesenchymal stem cells elicit polarization ofm2 macrophages and enhance cutaneous wound healing" (2010) Stem Cells 28:1856-1868; Fournier et al. "Multipotent progenitor cells in gingival connective tissue" (2010) Tissue Eng. Part A16:2891-2899; Marynka-Kalmani et al. "The lamina propria of adult human oral mucosa harbors a novel stem cell population" (2010) Stem Cells 28:984-995; Mitrano et al. "Culture and characterization of mesenchymal stem cells from human gingival tissue" (2010) J. Periodontol 81:917-925; Su et al. "Human gingiva derived mesenchymal stromal cells attenuate contact hypersensitivity via prostaglandin E(2)-dependent mechanisms" (2011) Stem Cells 29:1849-1860; and Tang et al. "Characterization of mesenchymal stem cells from human normal and hyperplastic gingiva" (2011) J Cell Physiol. 226:832-842.

Gingivae represent a unique oral tissue that serves as a biological mucosal barrier to protect the oral cavity side of maxilla and mandible. Gingiva is attached to the alveolar bone of tooth sockets. It is a distinct component of the oral mucosal immunity. Wound healing within the gingiva and oral mucosa is characterized by markedly reduced inflammation, rapid re-epithelialization and fetal-like scarless healing, contrary to the common scar formation present in skin. See, for example, Irwin et al. "Inter- and intrasite heterogeneity in the expression of fetal-like phenotypic characteristics by gingival fibroblasts: potential significance for wound healing" (1994) J. Cell Sci. 107 (Pt5): 1333-1346; Stephens et al. "Skin and oral fibroblasts exhibit phenotypic differences in extracellualr matrix reorganization and matrix metalloproteinase activity" (2001) Br. J. Dermatol. 144: 229-237. Such differences in wound healing between gingival/oral mucosa and skin may be attributed to the unique tolerogenic properties of the oral mucosal/gingival immune network. See, for example, Novak et al. "The immune privilege of the oral mucosa" (2008) Trends Mol. Med. 14: 191-198.

From a developmental point of view, craniofacial mesenchyme is derived from neural crest and mesoderm. For example, see Driskell et al. "Hair follicle dermal papilla cells at a glance" (2011) J. Cell Sc.i 124:1179-1182.

Cranial neural crest cells (CNCCs) migrate ventrolaterally as they populate the first branchial arches from the 4-somite stage, giving rise to mesenchymal structures, such as neural tissues, cartilage, bone, and teeth, in the craniofacial region. See for example, Chai et al. "Fate of the mammalian cranial neural crest during tooth and mandibular morphogenesis" (2000) Development 127:1671-1679; and Chai et al. "Recent advances in craniofacial morphogenesis" (2006) Dev. Dyn. 235:2353-2375.

Meanwhile, the mesoderm is also involved in orofacial development. Previous study showed the progenitor cells from oral mucasa lamina propria may be derived from neural crest cells. For example, see Davies et al. "A multipotent neural crest-derived progenitor cell population is resident within the oral mucosa lamina propria" (2010) Stem Cells Dev. 19:819-830.

In this context, WO 2013/076726 A1 describes methods and uses of neural cells differentiated from adult stem cells of the oral mucosa. Further, Tomar *et al.* (Tomar, G. B. et al.; Biochemical and Biophysical Research Communications, 393; 2010; pp. 377-383) describes human gingiva-derived mesenchymal stem cells (MSCs) in comparison to bone marrow-derived MSCs for cell therapy.

Although MSCs hold great promise for numerous medical applications, up until now, most stem cell therapies are based on well-characterized MSCs derived from bone marrows. Given that extracting stem cells from bone marrows is a difficult procedure with limited yield, this has placed a significant limitation on the development of their therapeutic applications. Recently, adipose stem cells have been investigated as a potential source of stem cells. However, while it is easier to extract adipose stem cells than bone marrow stem cells, the extraction process is still not yet perfected and the resulting stem cells are only suitable for a limited range of applications.

Therefore, there still exists a need for other sources of MSCs and new approaches for isolating thereof in order to have new and/or improved medical applications.

The present invention relates to the following items:
1. A method of preparing a composition suitable for a mesenchymal stem cell (MSC) treatment of a human, wherein the preparation method comprises:
   a. separating a gingival tissue obtained from a human into cells,
   b. sorting neural crest derived gingiva mesenchymal stem cells (N-GMSCs), wherein the sorting comprises sorting fluorescein isothiocyanate positive cells as N-GMSCs, and
   c. preparing a composition comprising N-GMSCs.
2. The preparation method of item 1, wherein the separating the gingival tissue into cells is done by a combination of a mechanical and a chemical method, wherein the chemical method is an enzymatic digestion method.
3. The preparation method of item 2, wherein the enzymatic digestion method comprises digesting the gingival tissue by using a solution comprising a collagenase and a dispase, and thereby obtaining a digested gingival tissue.
4. The preparation method of item 3, wherein the collagenase is collagenase type I and the dispase is dispase II.
5. The preparation method of item 3, wherein the separating the gingival tissue into cells further comprises preparing a cell suspension from the digested gingival tissue using a mechanical method.
6. The preparation method of item 5, wherein the mechanical method comprises filtering the digested gingival tissue to obtain the cell suspension.
7. The preparation method of item 6, wherein the cell suspension is a single-cell suspension.
8. The preparation method of item 5, wherein the preparation method further comprises culturing the separated cells before sorting N-GMSCs.
9. The preparation method of item 8, wherein culturing the separated cells comprises providing a solid surface, seeding the cells on the solid surface, culturing the seeded cells, and thereby obtaining a culture comprising cells that are adherent to the solid surface and cells that are not adherent to the solid surface.
10. The preparation method of item 9, wherein the method further comprises eliminating from the culture the cells that are not adherent to the solid surface.
11. The preparation method of item 10, wherein the method further comprises dissociating from the solid surface the cells that are adherent to the solid surface by using an enzyme.
12. The preparation method of item 9, wherein the method further comprises expanding the cultured cells.

This disclosure relates in general to a mesenchymal stem cell (MSC) therapy. This disclosure further relates to the isolation and applications of gingiva derived mesenchymal stem cells. More particularly, this disclosure relates to the isolation and applications of the neural crest derived gingiva mesenchymal stem cells.

Also described herein is a composition. This composition may comprise an isolated mesenchymal stem cell from neural crest cells. Neural crest cells may be cranial neural crest cells. Neural crest cells may be derived from gingiva. For example, the composition may comprise an isolated neural crest derived gingiva mesenchymal stem cell (N-GMSC). An isolated N-GMSC may be a cell that is fluorescein isothiocyanate (FITC) positive.

The composition may comprise an isolated N-GMSC that may have a capability to differentiate into neural crest cells and chondrocytes, and to induce activated T cell apoptosis.

The composition may comprise an isolated N-GMSC with immunomodulatory properties.

The composition may comprise an isolated mesenchymal stem cell from mesoderm. Mesoderm may be derived from gingiva. A mesenchymal stem cell from mesoderm may be a cell that is fluorescein isothiocyanate (FITC) negative.

The composition may comprise an isolated mesenchymal stem cell from neural crest cells and an isolated mesenchymal stem cell from mesoderm.

The composition may comprise an N-GMSC that may have an increased capacity to differentiate to neural cells as compared to a composition comprising an isolated mesenchymal stem cell from mesoderm.

The composition may comprise an N-GMSC that may have an increased capacity to differentiate to chondrocytes as compared to a composition comprising an isolated mesenchymal stem cell from mesoderm.

The composition may comprise an N-GMSC that may have an increased capacity to modulate immune cells as compared to a composition comprising an isolated mesenchymal stem cell from mesoderm.

Gingiva may be a gingiva of a mammal. The mammal may be a human. The mammal may be a non-human animal, such as a non-human primate, a horse, a sheep, a cattle, a hog, a dog, a cat, and a goat.

Also described herein is a method of preparation ("preparation method"). The preparation method may be a method of isolating N-GMSCs and/or M-GMSCs and/or preparing a composition using isolated N-GMSCs and/or M-GMSCs.

The preparation method may be a method of preparing a composition suitable for a MSC treatment of a mammal, wherein the preparation method may comprise: obtaining a gingival tissue, separating the gingival tissue into cells, sorting N-GMSCs, and preparing a composition comprising N-GMSCs. The preparation method may further comprise culturing the separated cells before sorting N-GMSCs. The sorting may comprise sorting fluorescein isothiocyanate positive cells as N-GMSCs.

The preparation method may also be a method of preparing a composition suitable for an MSC treatment of a mammal, wherein the preparation method may comprise: obtaining a gingival tissue, separating the gingival tissue into cells, culturing the separated cells, sorting fluorescein isothiocyanate positive cells from the cultured cells as N-GMSCs, and preparing a composition comprising N-GMSCs.

Gingiva may be a gingiva of a mammal. The mammal may be a human. The mammal may be a non-human animal, such as a non-human primate, a horse, a sheep, a cattle, a hog, a dog, a cat, and a goat.

The gingival tissue may be obtained from a mammal that undergoes the treatment. The gingival tissue may also be obtained from a mammal other than the mammal that undergoes the treatment. Combination of a said treatment methods may also be applied.

The separating the gingival tissue into cells may be done by a mechanical method, a chemical method, or a combination of a mechanical and a chemical method.

Examples of the chemical method may be digestion of the tissue by using acids, bases, and enzymes. For example, a collagenase and a dispase may be used to digest the tissue. The collagenase may be collagenase type I. The dispase may be the dispase II. A combination of these chemical methods may also be used to have separated cells.

The preparation method may further comprise preparing cell suspensions from the digested gingival tissue by using a mechanical method. An example of such method may be filtering the digested gingival tissue to obtain cell suspensions. The cell suspensions may be single-cell suspensions.

The culturing the separated cells may comprise providing a solid surface, seeding the cells on the solid surface, culturing the seeded cells, and thereby obtaining a culture comprising cells that may be adherent to the solid surface ("adherent cells") and cells that may not be adherent to the solid surface ("non-adherent cells").

The seeding the cells may be done in a solution. The solution may comprise a medium suitable for culturing the mammalian cell.

The preparation method may further comprise eliminating from the culture the cells that are not adherent to the solid surface.

The preparation method may further comprise dissociating from the solid surface the cells that are adherent to the solid surface. The adherent cells may be dissociated from the solid surface by using an enzyme. The enzyme, for example, may be trypsin.

The preparation method may further comprises expanding the cultured cells.

This disclosure also relates to a method of treating ("treatment method) of the mammal using a composition comprising N-GMSCs. The composition comprising N-GMSCs may be prepared according to the preparation method disclosed above.

The treatment method may comprise using the composition to regenerate neural tissue. The treatment method may comprise using the composition to repair damaged cartilage. The treatment method may comprise inducing activated T cell apoptosis.

The treatment method may comprise using the composition to heal a wound and/or to treat inflammatory and/or autoimmune diseases. The inflammatory and/or autoimmune diseases may be graft-versus-host disease (GvHD), diabetes, rheumatoid arthritis (RA), autoimmune encephalomyelitis, systemic lupus erythematosus (SLE), multiple sclerosis (MS), periodontitis, inflammatory bowel disease (IBD), alimentary tract mucositis induced by chemo- or radiotherapy, and sepsis.

The treatment method may comprise using the composition to reduce wrinkles, and/or for soft tissue augmentation and/or skin rejuvenation.

The treatment method may comprise using the composition to suppress peripheral blood lymphocyte proliferation or to induce the expression of immunosuppressive factors.

The treatment method may comprise using the composition to induce the expression of interleukin 10 (IL-10), indoleamine 2,3-dioxygenase (IDO), nitric oxide synthase (iNOS), and cyclooxygenase-2 (COX-2).

Any combination of inventive features disclosed above may be possible and thereby within scope of this disclosure. For example, the composition may be prepared by a method comprising: (a) obtaining a gingival tissue; (b) separating the gingival tissue into cells by digesting the cells and preparing a cell suspension from the digested cells; (c) culturing the separated cells comprising providing a solid surface, seeding the cells on the solid surface, culturing the seeded cells, and thereby obtaining a culture comprising cells that are adherent to the solid surface and cells that are not adherent to the solid surface; (d) eliminating from the culture the cells that are not adherent to the solid surface; (e) dissociating from the solid surface the cells that are adherent to the solid surface; and (f) sorting fluorescein isothiocyanate positive cells from the cultured cells as neural crest derived gingiva mesenchymal stem cells (N-GMSCs), and (g) preparing a composition comprising the N-GMSCs. The cells may be digested by using enzymes such as collagenase type I and dispase II. The adherent cells may be dissociated from the solid surface by using an enzyme like trypsin.

The drawings disclose illustrative embodiments. They do not set forth all embodiments. Other embodiments may be used in addition or instead. Details which may be apparent or unnecessary may be omitted to save space or for more effective illustration. Conversely, some embodiments may be practiced without all of the details which are disclosed. When the same numeral appears in different drawings, it refers to the same or like components or steps.

The figures show:

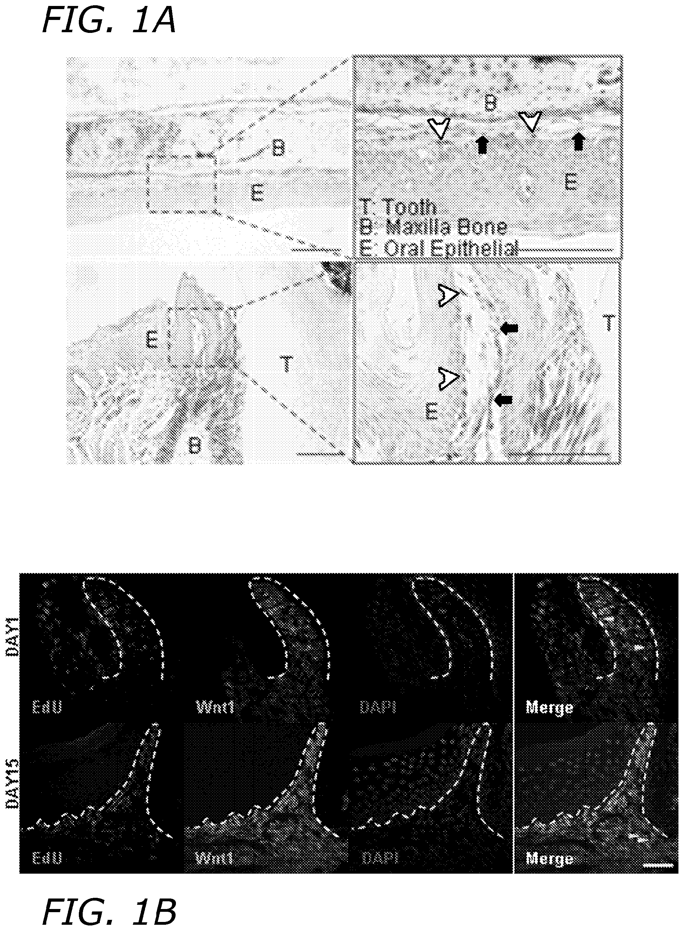

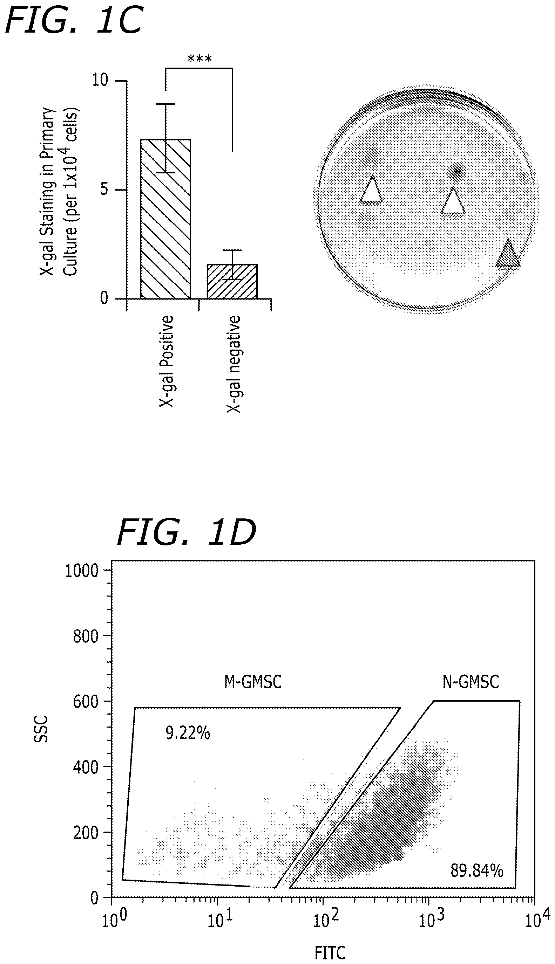

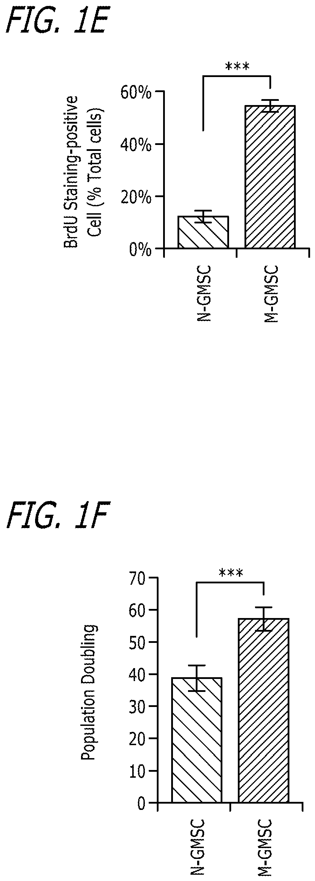

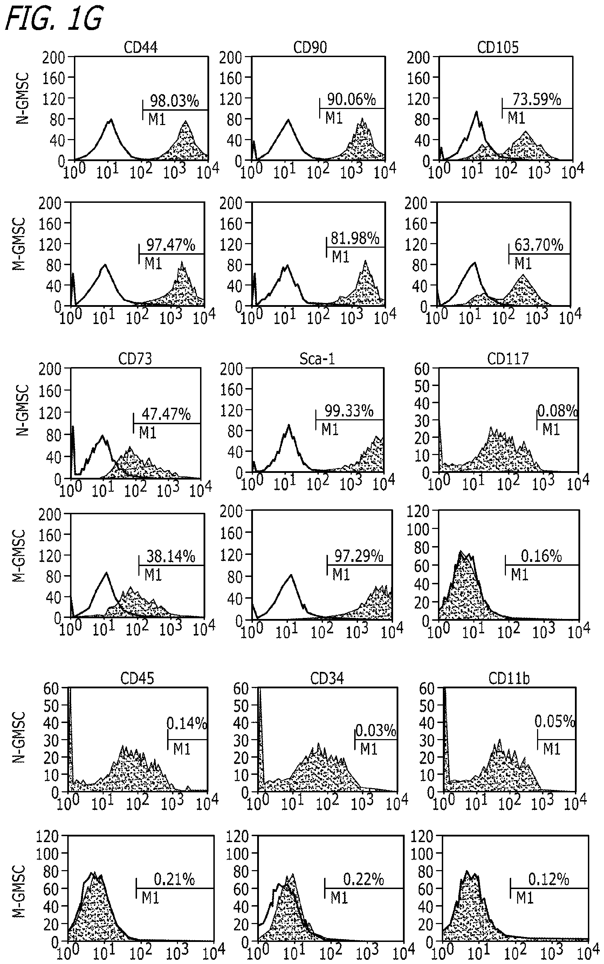

**FIG. 1****:** An exemplary characterization of Neural Crest Derived Gingiva Mesenchymal Stem Cells (N-GMSCs) and Mesoderm Derived Gingiva Mesenchymal Stem Cells (M-GMSCs). (A) X-gal staining showed that gingiva contained neural crest cell-derived cells (indicated by white arrows) and mesoderm-derived cells (indicated by black arrows); T: Tooth, B: Bone, E: Epithelial, scale bar = about 100 µm. (B) Injection (i.p.) of EdU (500 µg in 200 µl) in about 4-week-old Wnt1-Cre;Zsgreen mice. Maxilla samples were harvested on about day 1 and about day 15 post EdU injection. The majority of the EdU⁺ cells co-localized with the Zsgreen⁺ neural crest derived cells. Some EdU⁺ cells failed to co-localize with neural crest cells (white triangle). (White dotted line: epithelial basement line, scale bar = about 100µm). (C) Single colony assay showed that neural crest origin N-GMSCs (white arrows) and M-GMSCs (grey arrow) (n = 5). (D) N-GMSCs (FITC-positive) and M-GMSCs (FITC negative) were isolated from Wnt1-Cre; Zsgreen mice by flow cytometry. (E) Proliferation rate of cultured GMSCs was assessed by BrdU incorporation assay for about 24 hours. The number of BrdU-positive cells was indicated as a percentage of the total number of counted GMSCs and averaged from 5 replicated cultures (n = 5). (F) Continued culture assay showed that M-GMSCs had more elevated population doublings than N-GMSCs (n = 3). (G) Flow cytometric analysis showed that both N-GMSCs and M-GMSCs were positive for the surface molecules CD 44, CD90, CD 105, CD73, Sca-1, while they failed to express CD34, CD45, CD117 and CD11b. Error bars represent mean ± Standard Deviation (SD), ***p<0.001.

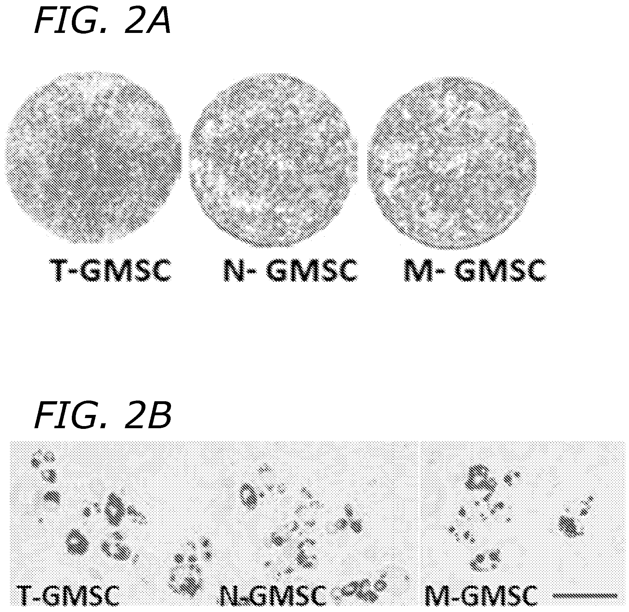

**FIG. 2****:** An example showing that (A) Alizarin red staining showed that N-GMSCs and M-GMSCs form similar amounts of mineralized nodules after culture in osteoinductive conditions for about 4 weeks. T-GMSCs: Total Gingiva Mesenchymal Stem Cells = N-GMSCs + M-GMSCs. (B) Oil red O staining showed that N-GMSCs and M-GMSCs had similar capacity to differentiate into adipocytes after culture under adipoinductive conditions for about 2 weeks. Scale bar = about 100µm.

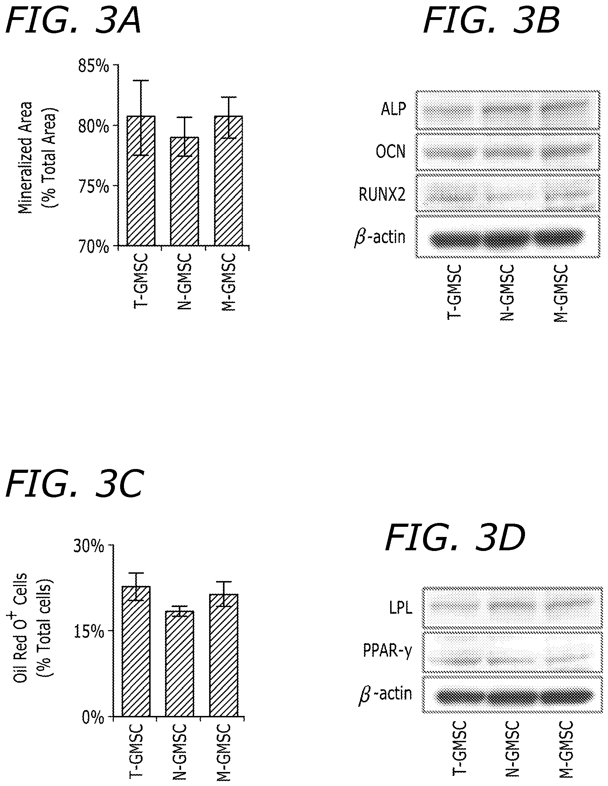

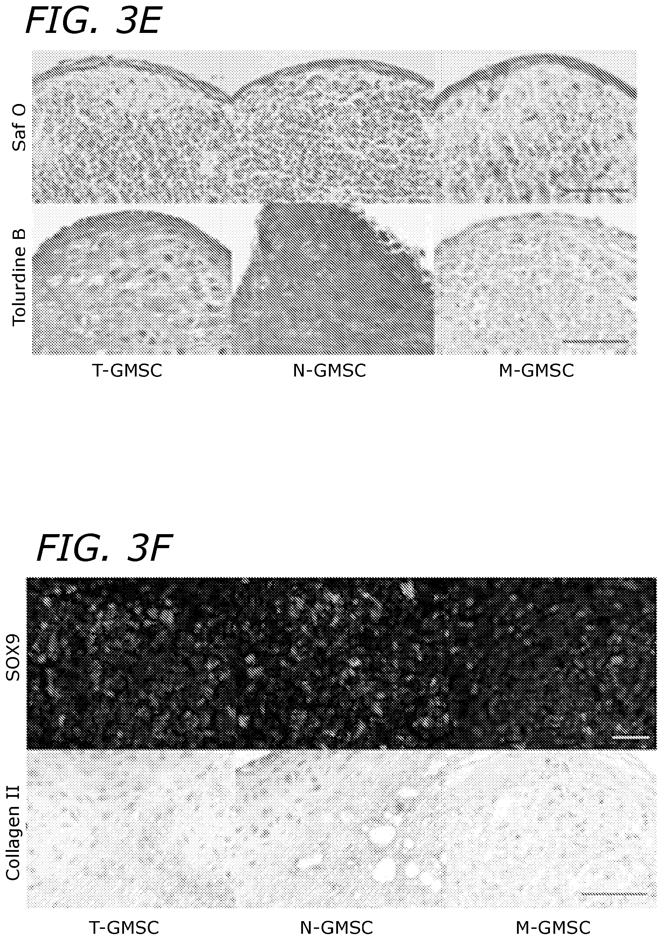

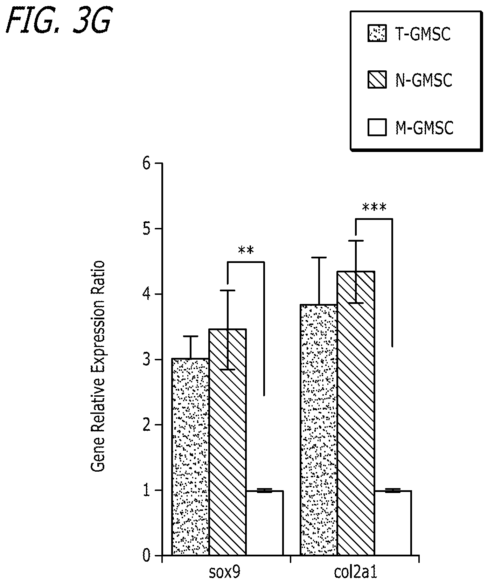

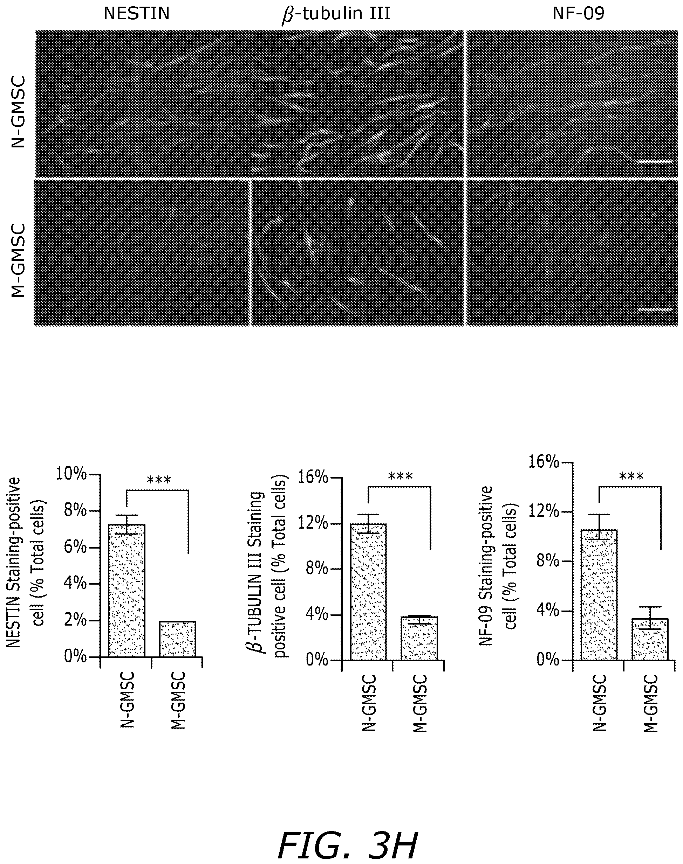

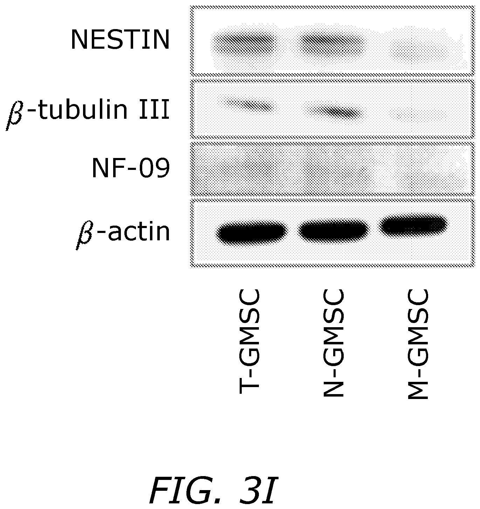

**FIG. 3****:** An exemplary multi-lineage differentiation of N-GMSCs and M-GMSCs. (A) Semiquantitative analysis of the percentage of alizarin red stained mineralized area versus total area after cultured in osteoinductive conditions for about 4 weeks indicated that there was no significant difference between N-GMSCs and M-GMSCs in forming mineralized nodules. (B) Western blot analysis confirmed that N-GMSCs and M-GMSCs expressed the same levels of the osteogenic genes, ALP, RUNX2, and OCN. Beta-actin, because of its stability, was used as a control to identify level of protein in each group. (C) No significant difference was seen between N-GMSCs and M-GMSCs in forming Oil red O-positive adipocytes after cultured in adipoinductive condition for about 2 weeks. (D) Western blot analysis confirmed that N-GMSCs and M-GMSCs expressed similar levels of the adipogenic markers PPARy-2 and LPL. Beta-actin, because of its stability, was used as a control to identify level of protein in each group. (E) Cell pellets derived from in vitro chondrogenic culture were sectioned and stained with safranin-O or toluidine blue. N-GMSCs showed more safranin-O-positive and toluidine blue-positive areas than the M-GMSC group. (F) Immunofluorescence staining showed that N-GMSCs expressed a higher level of SOX-9-positive cells when compared to M-GMSCs. Immunohistochemistry staining also showed stronger COLLAGEN II expression in the N-GMSC group. (G) Real-time PCR identified that N-GMSCs expressed higher sox9 and collagen II on the gene level when compared with M-GMSCs. (H) After culturing under the neural differentiation media for about 21 days, immunocytostaining showed that N-GMSCs had a significantly elevated expression of neurofilament M (NF-09), β-TUBULIN III, and NESTIN when compared to M-GMSCs (n = 5). (I) Western blot analysis confirmed that N-GMSCs expressed higher levels of neurogenesis protein NF-09, β-TUBULIN III and NESTIN compared with M-GMSCs. **p<0.01, ***p < 0.001, error bars: mean ± SD, scale bar = about 100µm.

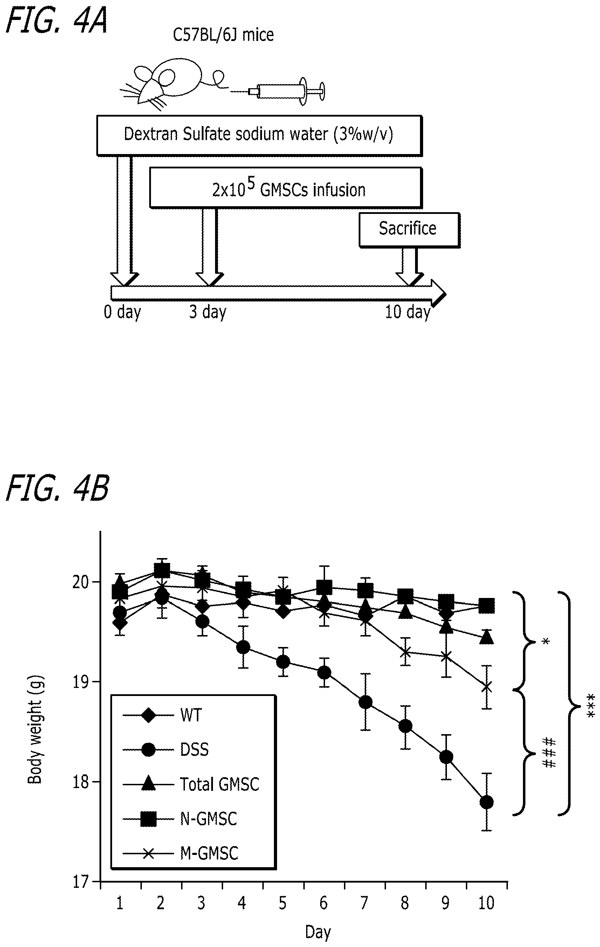

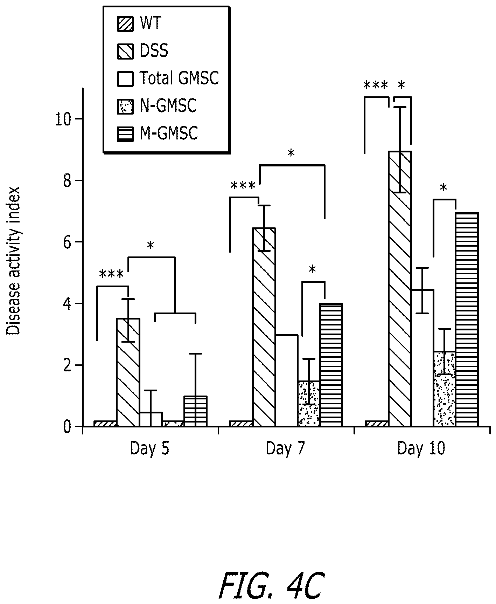

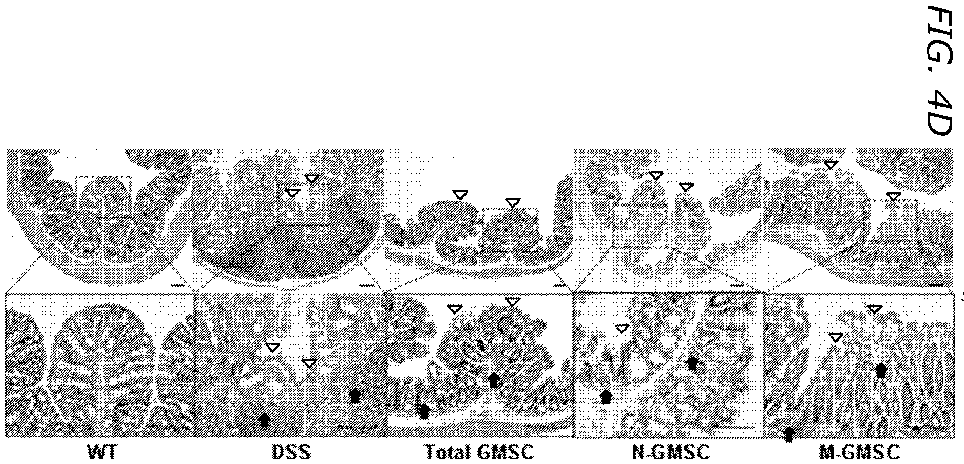

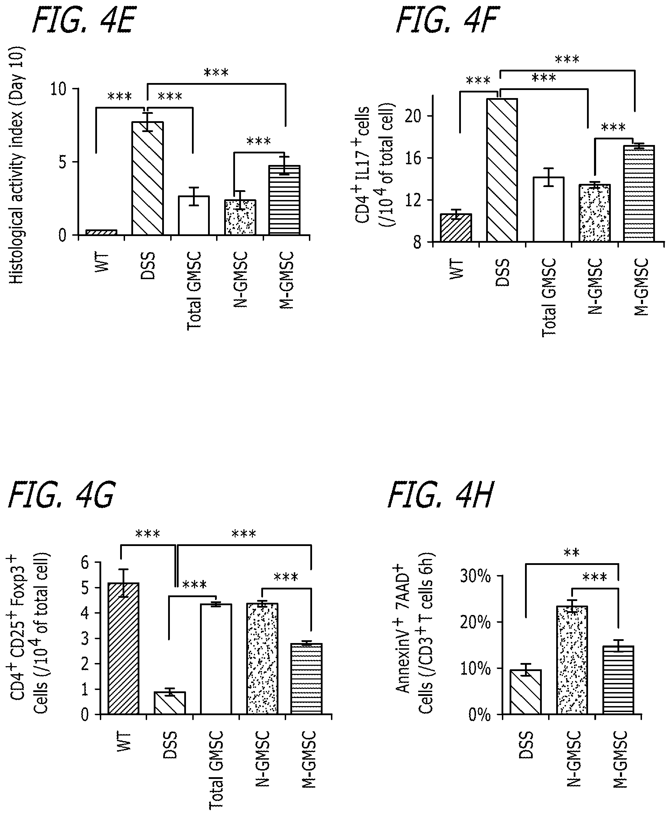

**FIG. 4****:** An example showing that N-GMSCs can ameliorate disease phenotype in dextran sulfate sodium (DSS)-induced experimental colitis. (A) Schema showing GMSCs infusion in DSS-induced experimental colitis mice. (B) Colitis mice (colitis, n = 5) showed significantly reduced body weight from about day 5 to about day 10 after DSS induction. N-GMSCs (n=5), M-GMSCs (n=5), and T-GMSCs (n=5) transplantation all reduced body weight loss compared to the colitis group (n=5) at about 10 days post-DSS induction. However, the N-GMSCs and T-GMSCs groups showed more significant reduction of body weight loss than the M-GMSCs group. (*p < 0.05 versus C57BL/6J; ***p < 0.001 versus C57BL/6J; ###p < 0.001 versus M-GMSCs). (C) Disease activity index (DAI) was significantly increased in colitis mice compared to C57BL/6J control mice (n=5) from about day 5 to about day 10 post-DSS induction. Although all GMSCs infusion groups showed significantly reduced DAI score, the N-GMSCs and T-GMSCs groups demonstrated superior reduction when compared to that of the M-GMSCs group. (D) Hematoxylin and Eosin (H&E) staining showed the infiltration of inflammatory cells (black arrows) in colon with destruction of epithelial layer (white triangles) in colitis mice. N-GMSCs transplantation exerted more significant rescue of disease phenotype in colon (D) and reduction of histological activity index (E) compared to the M-GMSCs group. (F) Th17 cell level was significantly elevated in colitis mice compared to C57BL/6J control mice at about 10 days post-DSS induction. Compared to the M-GMSC group, N-GMSC infusion showed a significant effect in reducing the levels of Th17 cells in colitis mice at about 10 days post-DSS induction. (G) Treg level was significantly reduced in colitis mice compared to C57BL/6J control mice at about 10 days post-DSS induction. N-GMSC infusion exhibited stronger capacity to upregulate the Treg levels in colitis mice compared with the M-GMSCs group. (H) By flow cytometric analysis, N-GMSC (n = 3) infusion showed marked elevation in the number of apoptotic CD3+ T cells compared to the M-GMSCs group at about 6 hours post-infusion in colitis mice. Scale bar = about 200 µm; *p < 0.05; **p<0.01; ***p < 0.001; error bars: mean ± SD.

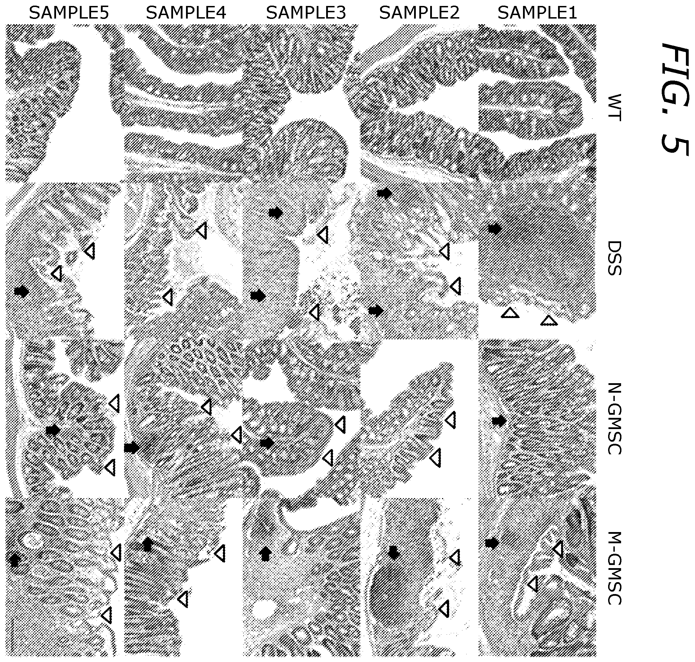

**FIG.5****:** An example showing histological examination of colon tissue. H&E staining showed that infiltration of inflammatory cell (black arrows) with destruction of epithelial layer (white triangles) in colons of colitis mice. N-GMSC transplantation resulted in a significant reduction of numbers of infiltration of inflammatory cells and destruction of epithelial layer compared to M-GMSC group. (Black arrows: lymphocytes infiltration; white triangles: epithelial margin; scale bar = about 200µm).

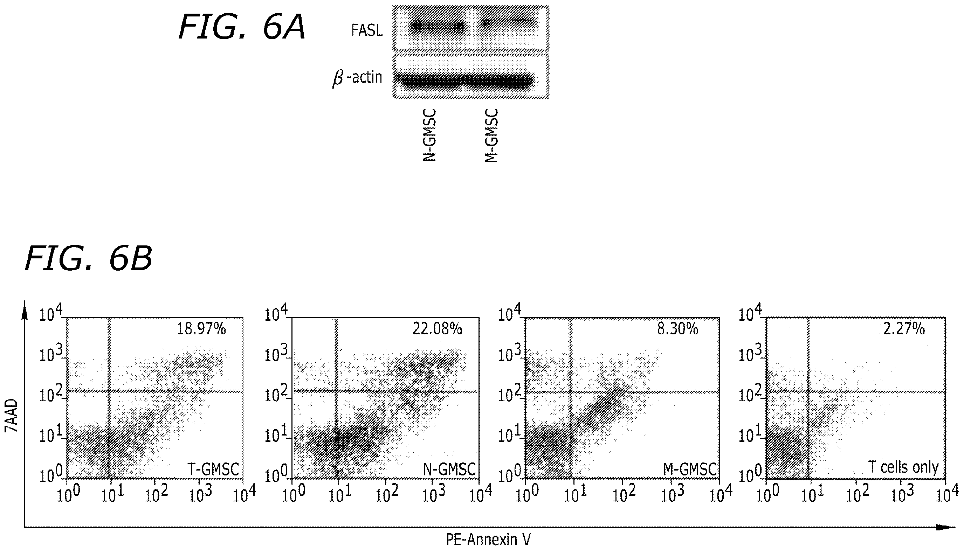

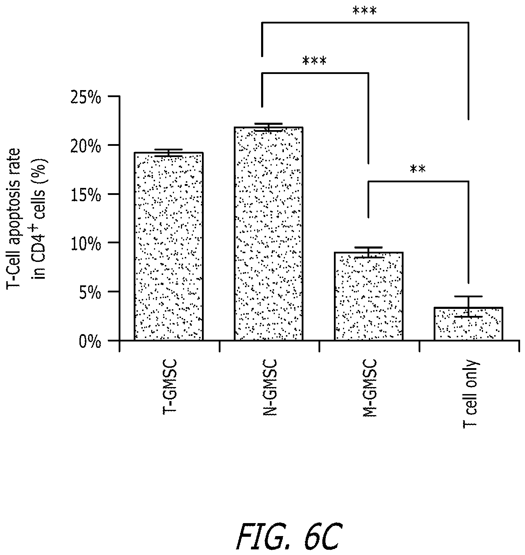

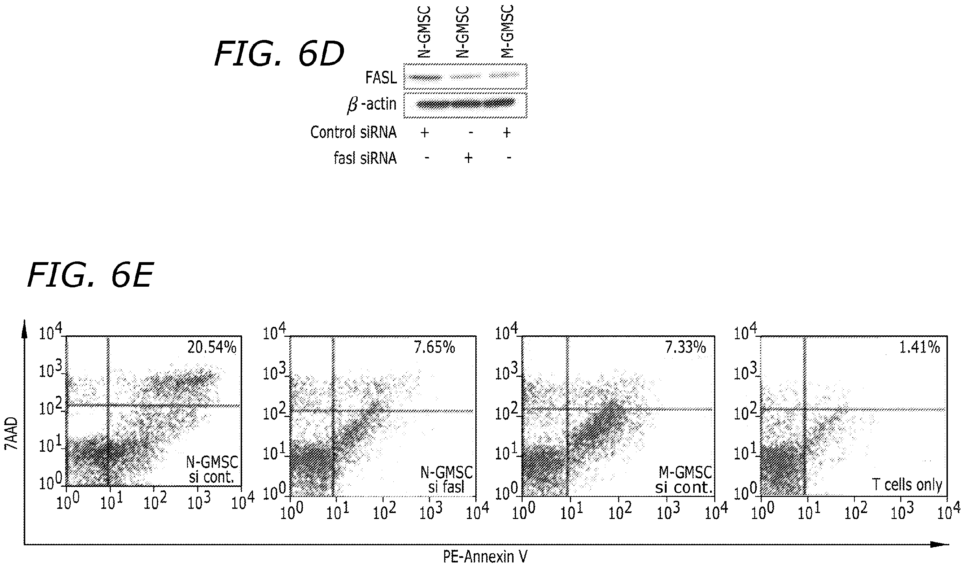

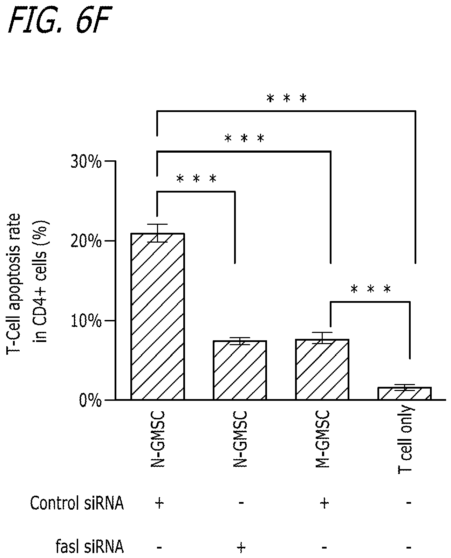

**FIG.6****:** An example showing that N-GMSCs require FAS Ligand (FASL) in order to maintain elevated immunomodulatory function. (A) Western blot analysis showed that N-GMSCs expressed elevated levels of FASL compared with M-GMSCs. (B, C) When co-cultured with T cells, N-GMSCs showed an elevated capacity to inhibit T cell viability as compared to M-GMSCs. Apoptosis assay confirmed that N-GMSCs more effectively induced 7AAD/Annexin-positive apoptotic T cells as compared to the M-GMSCs. (D) Western blot analysis showed efficacy of knockdown of FASL expression by siRNA in N-GMSCs. (E, F) When co-cultured with T cells, apoptosis assay confirmed that FASL knockdown N-GMSCs resulted in a reduced capacity to induce 7AAD/Annexin-positive apoptotic T cells when compared with the control siRNA group. **p < 0.01; ***p < 0.001; n = 3; error bars: mean ± SD.

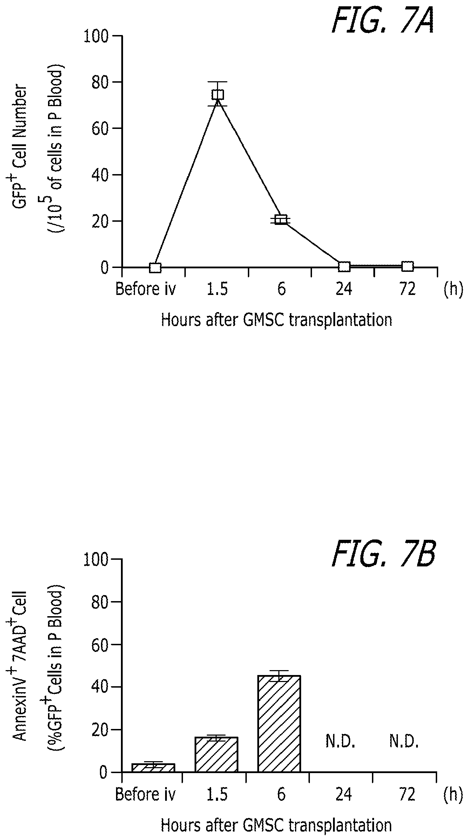

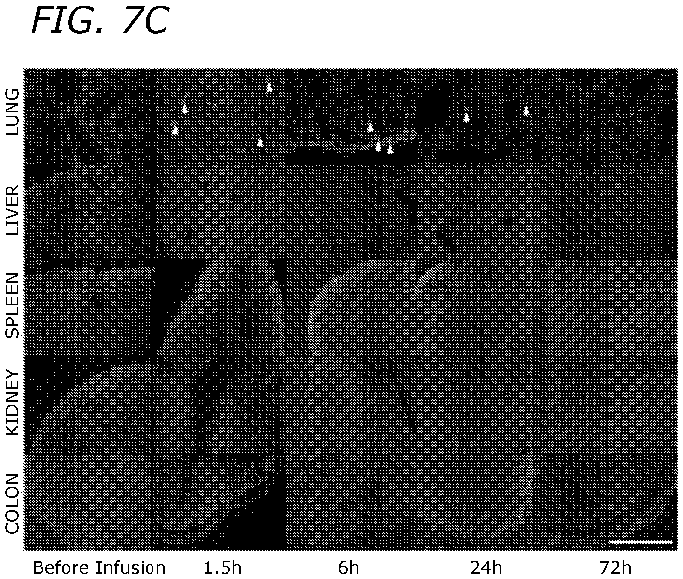

**FIG.7****:** An example showing that apoptosis of transplanted GMSCs in peripheral blood. (A) Flow cytometric analysis showed that the number of systemic infused GFP⁺ GMSCs reached a peak at about 1.5 hours post-transplantation in peripheral blood and then reduced to undetectable level at about 24 hours post-infusion. (B) The number of AnnexinV⁺7AAD⁺ double positive apoptotic GMSCs reached a peak at about 6 hours post-transplantation in peripheral blood and then reduced to undetectable level at about 24 hours post-transplantation. (C) The sections from lung, liver, spleen, kidney and colon at different time points. GFP⁺ cells (white triangles) can only be detected in lung during 1.5 hours - 24 hours post-transplantation. Error bars represent mean ± SD, scale bar = about 500µm).

Illustrative embodiments are now discussed. Other embodiments may be used in addition or instead. Details which may be apparent or unnecessary may be omitted to save space or for a more effective presentation. Conversely, some embodiments may be practiced without all of the details which are disclosed.

Following publication is mentioned herein: Xu et al. (2013) "Gingivae Contain Neural-crest- and Mesoderm-derived Mesenchymal Stem Cells" J Dent Res, 92(9):825-32.

Following acronyms were used.
5-FU: 5-fluorouracil
CNCC: Cranial Neural Crest Cell
COX-2: cyclooxygenase-2
DAI: disease activity index
DSS: Dextran Sulfate Sodium
FASL: FAS Ligand
FBS: Fetal bovine serum.
FITC: fluorescein isothiocyanate
GMSC: Gingiva Mesenchymal Stem Cell
GvHD: graft-versus-host disease
H&E: Hematoxylin and Eosin
HSCT: hematopoietic stem cell transplantation
IBD: inflammatory bowel disease
IDO: indoleamine 2,3-dioxygenase
IFN-γ interferon-γ
IL-10: interleukin 10
iNOS: inducible nitric oxide synthase
MS: multiple sclerosis
MSC: Mesenchymal Stem Cell
M-GMSC: Mesoderm Derived Gingiva Mesenchymal Stem Cell
NCC: Neural Crest Cell
N-GMSC: Neural Crest Derived Gingiva Mesenchymal Stem Cell
PBMC: peripheral blood mononuclear cells
PCR: Polymerase Chain Reaction
PD: Population doublings
RA: rheumatoid arthritis
SD: Standard Deviation
SLE: systemic lupus erythematosus
T-GMSC: Total Gingiva Mesenchymal Stem Cell = Neural Crest Derived Gingiva Mesenchymal Stem Cell + Mesoderm Derived Gingiva Mesenchymal Stem Cell.
µm: micrometer
α-MEM: α-Minimum Essential Media

This disclosure relates in general to a mesenchymal stem cell (MSC) therapy. This disclosure further relates to the isolation and applications of gingiva derived mesenchymal stem cells (GMSCs). More particularly, this disclosure relates to the isolation and applications of the neural crest derived gingiva mesenchymal stem cells (N-GMSCs).

The MSC therapy (or MSC treatment) includes, but not limited to, diagnosis, treatment, cure, healing, mitigation, or prevention of a disease or injury in, and/or cosmetic treatment of a mammal.

The gingiva may be a gingiva of any mammal. For example, the gingiva may be a gingiva of a mammal that undergoes the treatment (i.e. autologous stem cell treatment). Or, the gingiva may be a gingiva a mammal other than the mammal that undergoes the treatment (i.e. allogeneic stem cell treatment).

The mammal may be a human. The mammal may be a non-human animal, such as a non-human primate, a horse, a sheep, a cattle, a hog, a dog, a cat, and a goat.

This disclosure further relates to methods of using N-GMSCs for wound healing and/or to treat inflammatory and/or autoimmune diseases. Examples of such diseases are graft-versus-host disease (GvHD), diabetes, rheumatoid arthritis (RA), autoimmune encephalomyelitis, systemic lupus erythematosus (SLE), multiple sclerosis (MS), periodontitis, inflammatory bowel disease (IBD), alimentary tract mucositis induced by chemo- or radiotherapy, and sepsis. The other examples of method of using these GMSCs may be cosmetic injection to reduce wrinkles, soft tissue augmentation and other skin rejuvenation based on their ability to synthesize collagen, or any other applications of stem cells known in the art.

The N-GMSCs provided herein may have immunomodulatory and antiinflammatory properties. They may exhibit clonogenicity, self-renewal and multipotent differentiation capacities. For example, their immunomodulatory capabilities may include suppressing peripheral blood lymphocyte proliferation, inducing the expression of a wide panel of immunosuppressive factors including interleukin 10 (IL-10), indoleamine 2,3-dioxygenase (IDO), inducible nitric oxide synthase (iNOS), and cyclooxygenase-2 (COX-2) in response to the inflammatory cytokine, interferon-γ (IFN-γ).

These N-GMSCs may be easy to isolate and they may have an abundant tissue source. More importantly, their potentially rapid ex vivo expansion may render them an ideal source for stem cell-based therapeutic applications. Exemplary therapeutic methods may be systemic infusion, localized application, or other suitable means of formulation and delivery.

Isolation and application GMSCs were disclosed by Le et al. in a United States patent application publication, "Gingiva Derived Stem Cell and Its Application in Immunomodulation and Reconstruction" U.S. 2012/0128636 A1. Le et al. disclosed that GMSCs may be used to regulate inflammatory for wound healing and to treat inflammatory and/or autoimmune diseases.

Le et al. further disclosed an in vivo GMSC-based therapy using an established murine model of inflammatory disease, specifically inflammatory bowel disease (IBD).

Ulcerative colitis and Crohn's disease are two major forms of chronic inflammatory bowel disease (IBD) characterized by dysfunction of the innate and adaptive immunity, resulting in colonic mucosal injuries to the distal small intestine. See, for example, Podolsky "Inflammatory bowel disease" (2002) N. Engl. J. Med. 347: 417-429; and Xavier et al. "Unravelling the pathogenesis of inflammatory bowel disease" (2007) Nature 448: 427-434. There are several well-established murine models of human IBD. See, for example, Mizoguchi et al. "Inflammatory bowel disease, past, present and future: lessons from animal models" (2008) J. Gastroenterol. 43: 1-17. These animal models have provided useful tools for preclinical studies of therapeutic strategies, particularly stem cell-based therapies. See, for example, Gonza!ez et al. "Adipose-derived mesenchymal stem cells alleviate experimental colitis by inhibiting inflammatory and autoimmune responses" (2009) Gastroenterology 136: 978-989; Gonzalez-Rey et al. "Human adult stem cells derived from adipose tissue protect against experimental colitis and sepsis" (2009) GUT 58: 929-939; and Alex "Distinct cytokine patterns identified from multiplex profiles of murine DSS and TNBS-induced colitis" (2009) Inflamm. Bowel. Dis. 15: 341-352.

Le et al. also disclosed isolation and characterization of GMSCs from human gingival tissues. These GMSCs had multi-differentiation potential. These GMSCs were capable of suppressing human peripheral blood mononuclear cells (PBMCs) proliferation, indicating that they may have immunosuppressive effects.

Le et al. further disclosed a GMSC based therapy that ameliorated dextran sulfate sodium (DSS) induced colitis in mice. For the established murine model of colitis induced by oral administration of DSS, see, for example, Gonzalez-Rey et al. "Human adult stem cells derived from adipose tissue protect against experimental colitis and sepsis" (2009) GUT 58: 929-939; and Alex et al. "Distinct cytokine patterns identified from multiplex profiles of murine DSS and TNBS-induced colitis" (2009) Inflamm. Bowel. Dis. 15: 341-352. Le et al.'s results indicated that GMSC infusion had potential therapeutic effects in harnessing inflammation and reversing inflammatory-related tissue injuries.

Le et al. further disclosed that infusion of GMSCs is capable of reducing mucositis in 5-FU-induced alimentary tract mucositis in mouse models. Mucositis is the painful inflammation and ulceration of the mucous membranes lining the digestive tract, usually as an adverse effect of chemotherapy and radiotherapy treatment for cancer. Mucositis can affect up to 100% of patients undergoing high-dose chemotherapy and hematopoietic stem cell transplantation (HSCT), 80% of patients with malignancies of the head and neck receiving radiotherapy, and a wide range of patients receiving chemotherapy. For example, the commonly used anti-cancer agent, 5-fluorouracil (5-FU), leads to mucositis in up to about 40% of patients. Alimentary tract mucositis increases mortality and morbidity and contributes to rising health care costs. Le et al.'s results demonstrated that mucositis may be treated by infusion of GMSCs.

Le et al. also disclosed that wound healing may be enhanced by infusion of GMSCs.

In this disclosure, we showed that GMSCs (i.e. T-GMSC) may comprise neural crest derived gingiva mesenchymal stem cells (N-GMSCs) and mesoderm derived gingiva mesenchymal stem cells (M-GMSCs). We also showed that N-GMSCs may be isolated from the gingival tissue and/or isolated from GMSCs. We further showed that N-GMSCs may provide improved MSC therapy as compared to GMSCs and M-GMSCs.

N-GMSCs may be capable of clonogenicity, multiple differentiation capacity, and self-renewal. N-GMSCs may be capable of multiple differentiation into adipocytes, neural cells, endothelial cells, or osteoblasts.

Described herein is a composition. The composition may be a cell culture. The composition may be a drug or a biologic formulation. The composition may have immunomodulatory properties. The composition may be used in the treatment of a wound. The composition may be used in the treatment of a disease. The composition may comprise an isolated N-GMSC.

The disease may be an inflammatory disease. The disease may be an autoimmune disease. Examples of a disease may be graft-versus-host disease (GvHD), diabetes, rheumatoid arthritis (RA), autoimmune encephalomyelitis, systemic lupus erythematosus (SLE), multiple sclerosis (MS), periodontitis, inflammatory bowel disease (IBD), alimentary tract mucositis induced by chemo- or radiotherapy, and sepsis.

The composition may be used for neural tissue regeneration, cartilage repair, suppressing peripheral blood lymphocyte proliferation, and inducing the expression of an immunosuppressive factor. Examples of a immunosupressive factor may be interleukin 10 (IL-10), indoleamine 2,3-dioxygenase (IDO), inducible nitric oxide synthase (iNOS), and cyclooxygenase-2 (COX-2) in response to the inflammatory cytokine, and interferon-γ (IFN-γ).

N-GMSCs may be isolated from the gingiva. The gingiva may comprise neural crest cells and mesoderm. These neural crest cells may be cranial neural crest cells. N-GMSCs may be isolated from the neural crest cells of the gingiva. The isolated N-GMSC may be a cell that is fluorescein isothiocyanate (FITC) positive. The isolated N-GMSC may be a cell that has elevated capacity to differentiate into the neural crest cells. The isolated N-GMSC may be a cell that has elevated capacity to differentiate into chondrocytes. The isolated N-GMSC may be a cell that has elevated capacity to induce activated T cell apoptosis. The isolated N-GMSC may be a cell that has elevated capacity to differentiate into the neural crest cells and/or the chondrocytes, and/or to induce the activated T cell apoptosis.

M-GMSCs may be isolated from the gingiva. M-GMSCs may be isolated from the mesoderm of the gingiva. The isolated M-GMSC may be a cell that is fluorescein isothiocyanate (FITC) negative.

Also described herein is a method of preparation ("preparation method"). The preparation method may be a method of isolating N-GMSCs. The isolation method may be a method of preparing a composition using isolated N-GMSCs.

The preparation method may be a method of preparing a composition suitable for a MSC treatment of a mammal, wherein the preparation method may comprise: obtaining a gingival tissue, separating the gingival tissue into cells, sorting N-GMSCs, and preparing a composition comprising N-GMSCs. The preparation method may further comprise culturing the separated cells before sorting N-GMSCs. The sorting may comprise sorting fluorescein isothiocyanate positive cells as N-GMSCs.

The preparation method may also be a method of preparing a composition suitable for an MSC treatment of a mammal, wherein the preparation method may comprise: obtaining a gingival tissue, separating the gingival tissue into cells, culturing the separated cells, sorting fluorescein isothiocyanate positive cells from the cultured cells as N-GMSCs, and preparing a composition comprising N-GMSCs.

The gingival tissue may be a gingival tissue of a mammal. The mammal may be a human. The mammal may be a non-human animal, such as a non-human primate, a horse, a sheep, a cattle, a hog, a dog, a cat, and a goat.

The gingival tissue may be obtained from a mammal that undergoes the treatment. In this method, the treatment may be an autologous stem cell treatment. The gingival tissue may be obtained from a mammal other than the mammal that undergoes the treatment. In this method, the treatment may be an allogeneic stem cell treatment. Combination of said treatment methods may also be applied. For example, the treatment may comprise an autologous stem cell treatment and an allogeneic stem cell treatment.

The separating the gingival tissue into cells may be done by a mechanical method, a chemical method, or a combination of a mechanical and a chemical method.

Examples of the mechanical method may be mincing, shredding, filtering, and the like. In other examples, the gingival tissue may be separated into cells by using homogenizers, ultrasonicators, ball mills, and the like. A combination of these mechanical methods may also be used to have separated cells.

Examples of the chemical method may be digestion of the tissue by using acids, bases, and enzymes. For example, a collagenase and a dispase may be used to digest the tissue. The collagenase may be collagenase type I. The dispase may be dispase II. A combination of these chemical methods may also be used to have separated cells.

The preparation method may further comprise preparing cell suspensions from the digested gingival tissue by using a mechanical method. An example of such method may be filtering the digested gingival tissue to obtain cell suspensions. The cell suspensions may be single-cell suspensions. For example, single-cell suspensions may be obtained by passing the digested gingival tissue through a 70-µm strainer.

The culturing the separated cells may comprise providing a solid surface, seeding the cells on the solid surface, culturing the seeded cells, and thereby obtaining a culture comprising cells that may be adherent to the solid surface ("adherent cells") and cells that may not be adherent to the solid surface ("non-adherent cells").

The solid surface may be a surface of any solid article. For example, it may be a wall of a vessel. The vessel may be any vessel. For example, the vessel may be a petri dish or a cell-culture dish. The solid article may also be a bead or a particle. The solid article may have any size. For example, it may be a nano-particle.

The cell may be seeded using a solution. The solution may comprise a medium suitable for culturing the mammalian cell. An example of such medium may be α-MEM manufactured by Invitrogen (Carlsbad, CA). The solution may further comprise fetal bovine serum (FBS), L-glutamine, 2-mercaptoethanol, penicillin, and streptomycin.

The preparation method may further comprise eliminating from the culture the cells that are not adherent to the solid surface. For example, the culture may be washed by PBS to eliminate from the culture the cells that are not adherent to the solid surface.

The adherent cells may further be cultured, for example, in the same conditions disclosed above.

The preparation method may further comprise dissociating from the solid surface the cells that may be adherent to the solid surface. The adherent cells may be dissociated from the solid surface by using an enzyme. The enzyme, for example, may be trypsin.

The preparation method may further comprises expanding the cultured cells. For example, the expanding the cultured cells may comprise doubling N-GMSCs by repetitively re-seeding them using the preparation methods disclosed above.

This disclosure also relates to a method of treating ("treatment method) the mammal using a composition comprising N-GMSCs. The composition comprising N-GMSCs may be prepared according to the preparation method disclosed above.

The treatment method may comprise using the composition to regenerate neural tissue. The treatment method may comprise using the composition to repair damaged cartilage. The treatment method may comprise to induce activated T cell apoptosis.

The treatment method may comprise using the composition to heal a wound and/or to treat inflammatory and/or autoimmune diseases. The inflammatory and/or autoimmune diseases may be graft-versus-host disease (GvHD), diabetes, rheumatoid arthritis (RA), autoimmune encephalomyelitis, systemic lupus erythematosus (SLE), multiple sclerosis (MS), periodontitis, inflammatory bowel disease (IBD), alimentary tract mucositis induced by chemo- or radiotherapy, and sepsis.

The treatment method may comprise using the composition to reduce wrinkles, and/or for soft tissue augmentation and/or skin rejuvenation.

The treatment method may comprise using the composition to suppress peripheral blood lymphocyte proliferation or to induce the expression of immunosuppressive factors.

The treatment method may comprise using the composition to induce the expression of interleukin 10 (IL-10), indoleamine 2,3-dioxygenase (IDO), nitric oxide synthase (iNOS), and cyclooxygenase-2 (COX-2).

Also described herein is a method of treating an inflammatory and/or autoimmune disease in a subject, which may comprise: a) administering GMSCs into the subject; b) comparing the amount of inflammation at the affected organ or site in a control subject with the treated subject; and c) determining that the amount of inflammation in the subject given GMSCs is less than the amount of inflammation in the control subject is indicative of treating the inflammatory and/or autoimmune disease. Examples of the inflammatory response of this method may be normal or pathological wound healing, the inflammatory and/or autoimmune disease is graft-versus-host disease (GvHD), diabetes, rheumatoid arthritis (RA), autoimmune encephalomyelitis, systemic lupus erythematosus (SLE), multiple sclerosis (MS), periodontitis, inflammatory bowel disease (IBD), alimentary tract mucositis induced by chemo- or radiotherapy, or sepsis.

Any combination of inventive features disclosed above may be possible and thereby within scope of this disclosure. For example, the composition may be prepared by a method comprising: (a) obtaining a gingival tissue; (b) separating the gingival tissue into cells by digesting the cells and preparing a cell suspension from the digested cells; (c) culturing the separated cells comprising providing a solid surface, seeding the cells on the solid surface, culturing the seeded cells, and thereby obtaining a culture comprising cells that are adherent to the solid surface and cells that are not adherent to the solid surface; (d) eliminating from the culture the cells that are not adherent to the solid surface; (e) dissociating from the solid surface the cells that are adherent to the solid surface; and (f) sorting fluorescein isothiocyanate positive cells from the cultured cells as neural crest derived gingiva mesenchymal stem cells (N-GMSCs), and thereby preparing a composition comprising the N-GMSCs. The cells may be digested by using enzymes such as collagenase type I and dispase II. The adherent cells may be dissociated from the solid surface by using an enzyme like trypsin.

Other exemplary embodiments of this disclosure are as follows.

### EXAMPLE 1. MATERIALS AND METHODS

### Mice.

Female C57BL/6J mice and B6.Cg*-Gt(ROSA)26Sor^{tm6(CAG-ZsGreen1)Hze}*/J (ZsGreen) mice were purchased from the Jackson Laboratory (Bar Harbor, ME, USA). Wnt1-Cre transgenic line mice and the R26R conditional reporter (*LacZ*) mice were gifts from Dr. Yang Chai's laboratory at Herman School of Dentistry of University of Southern California. Mating *Wnt1-Cre*^{*+*/*-*} mice with *R26R*^{+/+} mice generated *Wnt1-Cre;R26R* mice (double transgenic). Mating *Wnt1-Cre*^{+/-} mice with *ZsGreen*^{+/+} mice generated *Wnt1-Cre;ZsGreen* mice (double transgenic). About eight weeks old mice were used at the experiments under the protocols approved by University of Southern California's Institutional Animal Care and Use Committee (IUCAC) (#10941 and 11141).

### Antibodies and Reagents.

Anti-runt-related transcription factor 2 (RUNX2) and -Osteocalcin (OCN) antibodies were purchased from Millipore (Billerica, MA, USA). Anti-alkaline phosphatase (ALP), -Nestin, -β-TUBULIN III, -Collagen II, -Sox9 and - Neurofilament Medium (NF-09) antibodies were purchased from Abcam (Cambridge, MA, USA). Anti-Sca-1-PE, -CD34-PE, -CD44-PE, -CD45-PE, - CD11b-PE, -CD73-PE, -CD117-PE, -CD4-PerCP, -CD25-APC, -IgG1-PE, -IgG2aPE, -IgG2b-PE, -CD3ε and -CD28 antibodies were purchased from BD Bioscience (San Jose, CA, USA). Anti-Foxp3-PE, -IL17-PE, -CD105-PE and -CD90-PE antibodies were purchased from eBioscience (San Diego, CA, USA). Purified anti-peroxisome proliferator-activated receptor γ (PPARγ), -lipoprotein lipase (LPL) and -FAS Ligand (FASL) antibodies, as well as secondary antibodies were purchased from Santa Cruz Biosciences (Santa Cruz, CA, USA). Anti-β actin antibody was purchased from Sigma (St. Louis, MO, USA). EdU imaging kit was purchased from Invitrogen (Carlsbad, CA, USA).

### Progenitor Cell Isolation and Culture.

GMSCs from Wnt1-Cre;R26R mice and Wnt1-Cre;ZsGreen mice were cultured as follows. Gingiva tissues from a mouse's mandibular molar region were gently separated, minced, and digested with solution containing about 2 mg/mL collagenase type I (Worthington Biochemical, Freehold, NJ, USA) and about 4 mg/mL dispase II (Roche Diagnostics, Indianapolis, IN, USA) in phosphate buffered saline (PBS) for about 1 hour at about 37 °C. Single-cell suspensions were obtained by passing the cells through a 70-µm strainer (BD Biosciences, Franklin Lakes, NJ, USA). All nucleated cells (ANC) were seeded at about 1 × 10⁶ into 100-mm culture dishes (Corning, NY, USA) with α-MEM (Invitrogen, Carlsbad, CA, USA) supplemented with about 20% FBS, about 2 mM L-glutamine (Invitrogen), about 55 µM 2-mercaptoethanol (Invitrogen), 100 U/mL penicillin, and 100 µg/mL streptomycin (Invitrogen), followed by an initial incubation for about 48 hours under about 37°C at about 5% CO₂. The cultures were washed with PBS twice to eliminate the non-adherent cells. Attached cells were cultured for another 12 days under same condition in the complete medium mentioned above.

### Colony-forming Units-Fibroblastic (CFU-F) Assay.

The CFU-F assay was performed as disclosed in a publication by Yamaza et al. "Mouse mandible contains distinctive mesenchymal stem cells" (2011) J. Dent. Res. 90:317-324. Briefly, independent ANCs (1-1.5 x 10⁵) isolated from gingivae were seeded on 60 mm culture plates (Corning). After about 14 days, the culture plates were stained with a mixture of about 0.1% toluidine blue (Merck, Darmstadt, Germany) and about 2% paraformaldehyde (PFA, Merck) solution. Colonies containing > 50 cells were counted as single colony clusters. The CFU-F count was performed in 5 independent samples per experimental group.

### Detection of β-galactosidase (lacZ) activities by X-gal staining.

The X-gal staining was carried out as follows. Cells or slides were fixed for about 20 minutes at room temperature with about 0.2% glutaraldehyde in PBS and washed three times in a rinse solution (about 0.005% Nonidet P-40 and about 0.01% sodium deoxycholate in PBS). Cells or slides were stained overnight at room temperature using a standard staining solution (about 5 mM potassium ferricyanide, about 5 mM potassium ferrocyanide, about 2 mM MgCl₂, about 0.4% X-gal in PBS), rinsed twice in PBS and post-fixed in about 3.7% formaldehyde. Sections and cells were then counterstained with Nuclear Fast Red (Sigma).

### Proliferation Assay.

Proliferation rates of GMSCs were assessed by bromodeoxyuridine (BrdU) staining kit (Invitrogen, Carlsbad, CA, USA), according to the manufacturer's protocols.

### Population Doublings (PD).

Method of PD is as follows. Multiple single colony-derived GMSCs were trypsinized and seeded at about 2 x 10⁵ cells in 35-mm dishes (Corning) in complete growth medium at the first passage. Cells were harvested and seeded at the same number when they reached confluence. Population doublings (PD) were calculated by the following formula: PD = log₂ (number of harvested cells/number of seeded cells). PD numbers were determined by cumulative addition of total numbers generated from each passage until cells ceased dividing. The PD assay was repeated with 3 independent isolated cells for each group.

### Cell Sorting.

GMSCs from Wnt1-Cre;ZsGreen mice at passage 2 (P2) were trypsinized and washed in PBS supplemented with 2% heat-inhibited FBS. Cells were then transferred to a 5 ml polystyrene tube (Falcon, Franklin Lakes, NJ, USA) and applied through FACSAria II (BD Biosciences, San Jose, CA, USA). All of the fluorescein isothiocyanate (FITC)-positive cells were collected as N-GMSCs, while the FITC-negative cells were collected as M-GMSCs.

### Flow Cytometric Analysis.

A quantity of about 2 x 10⁵ cells with about 1 µg antibody or isotype-matched IgG control were incubated for about 1 hour at about 4 °C. All samples were analyzed by using FACSCalibur (BD Biosciences, San Jose, CA, USA).

### Real-Time Polymerase Chain Reaction (PCR).

Real-time PCA was performed as follows. Total RNAs were isolated from different GMSCs by means of the TRIzol® Reagent (Life Technologies, Invitrogen). RNA samples (about 1 µg) were reverse-transcribed in a Reverse Transcription system (QIAGEN). Primers used were: (sox9) forward, 5'-TCGACGTCAATGAGTTTGACCA-3', and reverse, 5'-ATGCCGTAACTGCCAGTGTAGG-3'; (Col2a1) forward, 5'-GGGCTCCAATGATGTAGAGATG-3' and reverse, 5'-CCCACTTACCAGTGTGTTTCG-3' and (GAPDH as an internal control) forward, 5'-GAAGGTGAAGTTCGGAGTC-3', and reverse, 5'-GAAGATGGTGATGGGATTTC-3'.

### Immunohistochemistry.

Immunohistochemistry staining was performed as follows. Tissue sections were treated with about 0.3% hydrogen peroxide and about 0.1% sodium azide in PBS, pH about 7.2, for about 30 min, and incubated with indicated primary antibodies, overnight, at about 4 °C. After washing with PBS, the sections were immunostained using VECTASTAIN ABC kit (Vector, Burlingame, CA, USA) according to the manufacturer's instructions. Finally, samples were counterstained with hematoxylin.

### Immunofluorescence Staining.

Immunofluorescence staining was performed as follows. GMSCs at passage 3 were seeded on chamber slides (Nunc) for neurogenetic induction and then fixed with about 4% paraformaldehyde (PFA). Slides were incubated with normal serum, which was from the same species of secondary antibody. After blocking, the slides were first incubated with the specific antibodies overnight at about 4 °C, followed by incubation with Alexa Fluor® 488-conjugated secondary antibody (about 1:200, Invitrogen) for about 30 min at room temperature in dark. Finally, slides were mounted with VECTASHIELD ® Mounting Media (Vector Laboratories).

### Multi-lineage Differentiation Assay.

For in vitro differentiation assay, P2, GMSCs were cultured under osteogenic, adipogenic, chondrogenic and neurogenic conditions as follows.

For in vitro osteogenic assay, GMSCs (passage 2) were cultured to confluence and changed to an osteoinductive medium containing about 2 mM β-glycerophosphate (Sigma, St. Louis, MO, USA), about 100µM L-ascorbic acid 2-phosphate (Wako Pure Chemical Industries Ltd., Osaka, Japan), and about 10 nM dexamethasone (Sigma). After about 4 weeks of osteoinductive culture, calcium deposits were detected by staining with about 1% Alizarin Red (Sigma). The mineralized areas were quantified by ImageJ and shown as a percentage of Alizarin Red-positive area over the total area.

For in vitro adipogenic induction assay, GMSCs (passage 2) were cultured to confluence and then induced under adipogenic medium containing about 500 µM isobutyl-methylxanthine, about 60 µM indomethacin, about 0.5 µM hydrocortisone, and about 10 µM insulin for about 2 weeks. Cultures were then stained with about 0.3% Oil Red-O. The number of Oil-Red O-positive droplet-containing cells were counted and shown as a percentage of Oil-Red O-positive cells over total cells. Three independent experiments were performed for this assay.

For in vitro chondrogenic induction assay, GMSCs (passage 2) were induced by using the "pellet culture" technique as disclosed in a publication by Johnstone et al. "In vitro chondrogenesis of bone marrow-derived mesenchymal progenitor cells" (1998) Exp Cell Res 238: 265-272. Briefly, approximately about 1 x 10⁶ cells were placed in a 5-mL polypropylene tube (Falcon), centrifuged to pellet and cultured in complete culture medium until the pellet formed a round shape. Then, about 1 mL of chondrogenic medium containing DMEM (Gibco) with about 15% FBS, about 2 mM L-glutamine, about 1% ITS+ (BD Bioscience), about 100 nM dexamethasone, about 100 µM ascorbic acid, about 2mM sodium pyruvate, about 100 U/mL penicillin, and about 100 µg/mL streptomycin was added, freshly supplemented with about 10 ng/mL of transforming growth factor-β1 (TGF-β1). Medium was changed every 3-4 days for about 4 weeks. After about 4 weeks, the pellets were fixed in about 4% PFA, embedded in paraffin, and cut into about 6 µm sections. Chondrogenic differentiation was determined by staining with about 0.1% safranin-O (Sigma) and about 0.1% toluidine blue (Sigma) solution. Three independent experiments were performed for this assay.

For neuronal differentiation, GMSCs were seeded in 2-well chamber slides (Nunc) and cultured in DMEM/F12 (Invitrogen) supplemented with about 10% FBS, about 1 x N-2 supplement (Life Technologies), about 100 U/ml penicillin, about 100 µg/ml streptomycin, about 10 ng/ml fibroblast growth factor 2, and about 10 ng/ml epidermal growth factor (R&D Systems) and cultured for 14-21 days. Medium was changed with every 3-4 days.

### EdU Detection Staining.

EdU⁺ label retaining cells were detected on sections by EdU imaging kit according to the manufacturer's instruction.

### Western Blot Analysis.

Western blot analysis was performed as follows. Total protein was extracted with M-PER mammalian protein extraction reagent (Thermo, Rockford, IL, USA). About 20 µg of protein were applied and separated on 4-12% NuPAGE gel (Invitrogen), followed by transfer to Immobilon™-P nitrocellulose membranes (Millipore Inc., Billerica, MA, USA). Membranes were blocked with about 5% non-fat dry milk and about 0.1% Tween-20 for about 1 hour, followed by incubation with the primary antibodies (about 1:200-1,000 dilution) at about 4°C overnight. Horseradishperoxidase-conjugated secondary antibody (Santa Cruz Biosciences; about 1:10,000) was used to treat the membranes for about 1 hour, followed by enhancement with a SuperSignal®West Pico Chemiluminescent Substrate (Thermo). Bands were detected on BioMax MR film (Kodak, Rochester, NY, USA). Each membrane was also stripped with a stripping buffer (Thermo) and reprobed with anti-P-actin antibody to quantify the amount of loaded protein.

### FASL Knockdown.

In order to knock down FASL expression, about 0.2 x 10⁶ GMSCs were seeded in a 12-well culture plate. FASL siRNA (Santa Cruz Biotechnology, Santa Cruz, CA, USA) was used according to the manufacturer's protocols.

### Co-culture of GMSCs with Activated Splenocytes.

Co-culture of GMSCs with activated splenocytes was performed as follows. After collection of spleen from mice, red blood cells were removed with ACK lysis buffer (Gibco, Grand Island, NY, USA), and the splenocytes (2-3 x 10⁶) were activated with plate-bound about 1 mg/mL anti-CD3ε antibody and about 1 mg/mL soluble anti-CD28 antibody (BD) for about 3 days in complete medium containing Dulbecco's Modified Eagle's Medium (DMEM, Lonza, Basel, Switzerland) with about 10% heat-inactivated FBS, about 50 µM 2-mercaptoethanol, about 10 mM HEPES, about 1 mM sodium pyruvate (Sigma), about 1% non-essential amino acid (Cambrex), about 2 mM L-glutamine, about 100 U/mL penicillin, and about 100mg/mL streptomycin.

Seeding of about 2 x 10⁵ mouse GMSCs to a 12-well dish was followed by adding activated splenocytes (about 1 x 10⁶/well) to co-culture for about 3 days. To measure T cell viability, flow cytometry was used to detect T cell apoptosis by Annexin V-PE Apoptosis Detection Kit (BD Pharmingen).

### Dextran Sulfate Sodium (DSS)-induced Mouse Colitis and Treatment with GMSCs.

Acute colitis was induced in C57BL/6J mice by administering about 3% (w/v) DSS (molecular mass 36-50 kDa; MP Biochemicals, Solon, OH, USA) in drinking water for about 10 days, as disclosed in a publication by Zhang et al. "Mesenchymal stem cells derived from human gingiva are capable of immunomodulatory functions and ameliorate inflammation-related tissue destruction in experimental colitis" (2009) J. Immunol. 183:7787-7798. After sorting by flow cytometry, about 2 × 10⁵ GMSCs were re-suspended in PBS and infused into the colitis mice (n = 5 per group) intravenously at about day 3 post-DSS feeding. At about day 10, peripheral blood was collected for Th17, Treg level assay. Then the mice were euthanized, and entire colon was collected and gently cleared of feces with sterile PBS. For histopathological analysis, colon segments were fixed in about 4% PFA, and paraffin-embedded sections were prepared for H&E staining.

For GMSC treatment, about 0.2 x 10⁵ of P2 GMSCs were infused into the colitis mice (n = 5 each group) intravenously at about 3 days post DSS-induction. All mice were euthanized at about day 10 and analyzed, as described in a publication by Alex et al. "Distinct cytokine patterns identified from multiplex profiles of murine DSS and TNBS-induced colitis" (2009) Inflamm. Bowel. Dis. 15:341-352. The results are representative of 3 independent experiments.

### Statistics

Statistical analysis was performed by using SPSS 13.0. Significance was assessed by independent two-tailed Student's t-test or analysis of variance (ANOVA). P values less than 0.05 were considered as significant.

### EXAMPLE 2. CHARACTERIZATION OF N-GMSCS AND M-GMSCS

N-GMSCs and M-GMSCs were characterized as follows. X-gal staining showed that gingiva mesenchyme of Wnt1-Cre;R26R (LacZ) mice contained CNCC-derived β-galactosidase-positive cells and mesoderm-derived β-galactosidase-negative, but Nuclear Fast Red-positive cells, as shown in **FIG.1A****.** When EdU was injected (i.p.) into Wnt1-Cre; Zsgreen mice for about 7 days and traced for about 2 weeks, majority of the EdU⁺ cells co-localized with the Zsgreen⁺ neural crest derived cells. Some EdU⁺ cells failed to co-localize with neural crest cells (white triangle), as shown in **FIG.1B****.** When isolating MSCs from the gingiva and culturing at a low density, most adherent single colony clusters were found to be β-galactosidase-positive N-GMSCs with fewer β-galactosidase-negative M-GMSCs clusters, as shown in **FIG.1C****.** Next, we used Wnt1-Cre;Zsgreen mice, in which CNCC-derived cells continuously express ZsGreen protein and are FITC-positive under flow cytometric analysis to accurately separate N-GMSCs and M-GMSCs. We found that about 90% of single colony-derived GMSCs were N-GMSCs, while about 10% were M-GMSCs, as shown in **FIG.1D****.** M-GMSCs showed an elevated cell proliferation rate and population doubling, as determined by BrdU incorporation and continued culture assays, respectively, when compared to N-GMSCs, as shown in **FIG.1E-F****.** Flow cytometric analysis confirmed that both N-GMSCs and M-GMSCs were positive for the mesenchymal stem cell surface markers CD44, CD90, CD105, CD73 and Sca-1, but negative for the hematological markers CD117, CD45, CD34 and the macrophage marker CD11b, as shown in **FIG.1G****.**

### EXAMPLE 3. MULTI-LINEAGE DIFFERENTIATION OF N-GMSCS AND M-GMSCS

Under the osteogenic culture conditions, N-GMSCs and M-GMSCs showed the same capability to form mineralized nodules, as shown in **FIG.2A****,** as assessed by Alizarin Red staining, and express the osteogenic markers alkaline phosphatase (ALP), osteocalcin (OCN) and runt-related transcription factor 2 (RUNX2), as determined by Western blot analysis, as shown in **FIG.3A-B****.** Also, N-GMSCs and M-GMSCs had the same adipogenic differentiation potential, as assessed by Oil red O-positive staining, as shown in **FIG.2B****,** to show the number of adipocytes and Western blot to show expression of the adipocyte-specific transcripts peroxisome proliferator-activated receptor γ (PPAPγ) and lipoprotein lipase (LPL), as shown in **FIG.3C-D****.** To assess chondrogenic capacity, N-GSMCs and M-GMSCs were cultured in chondrogenic induction media for four weeks. Safranin-O and toluidine blue staining showed that N-GMSCs generated more cartilage matrix than M-GSMCs, as shown in **FIG.3E****.** Additionally, immunofluorescence and immunohistochemistry staining showed that N-GMSCs express a high level of the chondrogenic marker SOX9 and Collagen II compared to M-GMSCs, as shown in **FIG.3F****.** Real-time PCR identified that N-GMSCs expressed higher sox9 and collagen II on the transcriptional level when compared with M-GMSCs, as shown in **FIG.3G****.**

Since the N-GMSCs were derived from CNCCs, we examined the neural differentiation potential of N-GMSCs and revealed that N-GMSCs showed significantly elevated expression of neural makers, including neurofilament M (NF-09), β-TUBULIN III and NESTIN, when cultured under neural induction condition for about 3 weeks in comparison to M-GSMCs, as shown in **FIG.3H****.** Western blot analysis confirmed that N-GMSCs expressed higher levels of neurofilament M (NF-09), β-TUBULIN III and NESTIN when compared with M-GMSCs, as shown in **FIG.3I****.**

### EXAMPLE 4. IMMUNOMODULATORY PROPERTY OF GMSCs

GMSCs may have an immunomodulatory property, as disclosed in a publication by Zhang et al. "Mesenchymal stem cells derived from human gingiva are capable of immunomodulatory functions and ameliorate inflammation-related tissue destruction in experimental colitis" (2009) J. Immunol. 183:7787-7798.

In this example, we compared immunotherapeutic effect of N-GMSCs to that of M-GSMCs in dextran sulfate sodium (DSS)-induced experimental colitis mice. This type of mice was disclosed in a publication by Alex et al. "Distinct cytokine patterns identified from multiplex profiles of murine DSS and TNBS-induced colitis" (2009) Inflamm. Bowel. Dis. 15:341-352.

N-GMSCs or M-GMSCs (2×10⁵) sorted by flow cytometry were systemically transplanted into experimental colitis mice at about day 3 after about 3% DSS treatment, as shown in **FIG.4A****.** Body weight of colitis mice was significantly reduced when compared to the control C57BL/6J mice, as shown in **FIG.4B****.** Although infusion of both N-GMSC and M-GMSC could partially rescue reduced body weight in DDS-induced colitis mice, N-GMSCs showed more significant rescue of reduced body weight compared to M-GMSCs, as shown in **FIG.4B****.** The disease activity index (DAI), including body weight loss, diarrhea, and bleeding, was significantly elevated in the colitis mice compared to the control group. After N-GMSC and M-GMSC infusion, the DAI score was decreased. However, N-GMSC infusion induced a more significant reduction in the DAI score than that of the M-GMSC group from about 5 days to about 10 days post-GMSC infusion, as shown in **FIG.4C****.** Colon tissues from each group were analyzed by histological section. Absence of epithelial layer and infiltration of inflammatory cells were observed in the colitis mice, but infusion of either N-GMSC or M-GMSC rescued impaired histological structures, as shown in **FIG.4D** and **FIG.5****.** Compared to the M-GMSC group, however, the N-GMSC group had superior histological recovery of the epithelial structure (yellow triangle) and elimination of inflammatory cells (blue arrow) in the colitis mice, as assessed by histological activity index, as shown in **FIG.4E****,** which included ameliorating colonic transmural inflammation, reducing wall thickness, suppressing epithelial ulceration, and restoring normal intestinal architecture. Reduced Tregs and elevated Th17 cells were observed in the colitis mice at about day 10 post-DSS induction, as shown in **FIG.4F-G****.** Both N-GMSC and M-GMSC transplantation upregulated Treg level, but downregulated the level of Th17 cells. However, the N-GMSC group showed more significant upregulation of Tregs and downregulation of Th17 cells when compared to the M-GMSC group, as shown in **FIG.4F-G****.**

It was previously disclosed that mesenchymal stem cell-induced T cell apoptosis could trigger macrophages to produce high levels of TGFβ, which, in turn, may lead to the up-regulation of CD4⁺CD25⁺Foxp3⁺ Tregs to result in immune tolerance. See the publication by Akiyama et al. "Mesenchymal-stem-cell-induced immunoregulation involves FAS-ligand-/FAS-mediated T cell apoptosis" (2012) Cell Stem Cell 10:544-555.

In this example, we showed that N-GMSC transplantation induced more marked T cell apoptosis than that achieved by the M-GMSC group, as shown in **FIG.4H****.**

### EXAMPLE 5. N-GMSC-MEDIATED IMMUNOMODULATION IS ASSOCIATED WITH ELEVATED EXPRESSION OF FAS LIGAND (FASL).

FASL may play an important role in mesenchymal stem cell-induced immune tolerance, as disclosed in a publication by Akiyama et al. "Mesenchymal-stem-cell-induced immunoregulation involves FAS-ligand-/FAS-mediated T cell apoptosis" (2012) Cell Stem Cell 10:544-555; and in a publication by Yamaza et al. "Immunomodulatory properties of stem cells from human exfoliated deciduous teeth" (2010) Stem Cell Res. Ther. 1:5-14.

This example demonstrates that N-GMSCs expressed an elevated level of FASL compared with M-GMSCs, as shown in **FIG.6A****.** To confirm that FASL expression was correlated with elevated immunomodulatory capacity in N-GMSCs, we first showed that N-GMSCs had significantly elevated capacity to induce activated T cell apoptosis in an in vitro co-culture system when compared to M-GMSCs, as shown in **FIG.6B-C****.** Then we used the siRNA approach to knock down FASL expression in N-GMSCs, as shown in **FIG.6D****,** and found that the capacity to induce activated T cell apoptosis by FASL siRNA-treated N-GMSCs was significantly reduced, as shown in **FIG.6E-F****.** When systemic infusion of GFP⁺ GMSCs, GFP⁺ cells reached the peak in peripheral blood at about 1.5 hours post-infusion, and became undetectable at about 24 hours, as shown in **FIG.7A****;** while GFP⁺ apoptotic cells reached the peak at about 6 hours post-infusion and became undetectable at about 24 hours post-infusion in peripheral blood, as shown in **FIG.7B****.** Immunostaining showed that only few GFP⁺ cells were detected in lung, but not in liver, spleen, kidney, and colon, at about 24 hours post-infusion, as assessed in multiple tissue sections, as shown in **FIG.7C****.** These data indicated that homing of infused GMSCs may not play a major role in GMSC-mediated therapy in the colitis mice.

The vertebrate neural crest cells (NCC) are multipotent cell population derived from the lateral ridges of the neural plate and gives rise to multiple types of derivatives, as disclosed in a publication by Bronner-Fraser et al. "Cell lineage analysis reveals multipotency of some avian neural crest cells" (1988) Nature 335:161-164. Some of postmigratory NCCs also possess the capacity for self-renewal and multipotent differentiation, as disclosed in publications by Chung et al. "Stem cell property of postmigratory cranial neural crest cells and their utility in alveolar bone regeneration and tooth development" (2009) Stem Cells 27:866-877; and Lo et al., "Postmigratory neural crest cells expressing c-RET display restricted developmental and proliferative capacities" (1995) Neuron 15:527-539. X-gal positive cells were found in the gingiva area of Mesp1-Cre;R26R mice, suggesting mesoderm-derived cells may contribute to gingivae formation, as disclosed by Rothová et al. "Contribution of mesoderm to the developing dental papilla" (2011) Int J Dev Biol 55:59-64. Temporomandibular joint cartilage may be developed from neural crest cells, as disclosed in a publication by Chai et al. "Fate of the mammalian cranial neural crest during tooth and mandibular morphogenesis" (2000) Development 127:1671-1679.

In Examples 1-5, we demonstrated that both NCC-derived stem cells and none-NCC-derived mesoderm stem cells reside in gingival mesenchyme. Although N-GMSCs and M-GMSCs showed some identical stem cell properties, such as expression of mesenchymal stem cell surface markers and multipotent differentiation, they also exhibited distinctive characteristics. N-GMSCs had significantly increased capacity to differentiate into neural cells when cultured under neural differentiation condition, suggesting the potential for their use in neural tissue regeneration. Also, N-GMSCs had greater potential to differentiate into chondrocytes when compared to M-GMSCs. The use of N-GMSCs for cartilage repair may be an optimal approach, especially for temporomandibular joint cartilage.

The FASL/FAS pathway is an important cell death pathway in many cell types, as disclosed by O'Reilly et al."Membrane-bound FAS ligand only is essential for FAS-induced apoptosis" (2009) Nature 461:659-663. Mesenchymal stem cell-induced immune tolerance was associated with FASL-triggered T cell apoptosis via the FAS pathway and macrophages subsequently took apoptotic T cells to release a high level of TGFb to up-regulate Tregs, as disclosed by Akiyama et al. "Mesenchymal-stem-cell-induced immunoregulation involves FAS-ligand-/FAS-mediated T cell apoptosis" (2012) Cell Stem Cell 10:544-555.

In Examples 1-5, it was demonstrated that N-GMSCs showed elevated capacity to ameliorate disease phenotype in DSS-induced colitis mice by the highly expressed FASL, which induced activated T cell apoptosis and eventually may result in immune tolerance. Furthermore, N-GMSCs possessed superior immunoregulatory function by expression of a high level of FASL. Conversely, knockdown of FASL in N-GMSCs showed a significant reduction in their immunoregulatory capacity.

The craniofacial facial region undergoes a unique development process compared with other parts of the body. Interplays between cells from different tissue layers may contribute to tissue development and formation. Epithelium of the middle ear has a dual origin, as disclosed in a publication by Thompson et al. "Dual origin of the epithelium of the mammalian middle ear" (2013) Science 339:1453-1456.

In sum, there was difference between N-GMSCs and M-GMSCs in the gingiva, as results of Examples 1-5 indicated. GMSCs comprised neural crest cell-derived N-GMSCs and mesoderm-derived M-GMSCs. In comparison to M-GMSCs, N-GMSCs showed an increased capacity to differentiate to neural cells and chondrocytes, as well as modulate immune cells.

## Claims

1. A method of preparing a composition suitable for a mesenchymal stem cell (MSC) treatment of a human, wherein the preparation method comprises:
a. separating a gingival tissue obtained from a human into cells,
b. sorting neural crest derived gingiva mesenchymal stem cells (N-GMSCs), wherein the sorting comprises sorting fluorescein isothiocyanate positive cells as N-GMSCs, and
c. preparing a composition comprising N-GMSCs.

2. The preparation method of claim 1, wherein the separating the gingival tissue into cells is done by a combination of a mechanical and a chemical method, wherein the chemical method is an enzymatic digestion method.

3. The preparation method of claim 2, wherein the enzymatic digestion method comprises digesting the gingival tissue by using a solution comprising a collagenase and a dispase, and thereby obtaining a digested gingival tissue.

4. The preparation method of claim 3, wherein the collagenase is collagenase type I and the dispase is dispase II.

5. The preparation method of claim 3, wherein the separating the gingival tissue into cells further comprises preparing a cell suspension from the digested gingival tissue using a mechanical method.

6. The preparation method of claim 5, wherein the mechanical method comprises filtering the digested gingival tissue to obtain the cell suspension.

7. The preparation method of claim 6, wherein the cell suspension is a single-cell suspension.

8. The preparation method of claim 5, wherein the preparation method further comprises culturing the separated cells before sorting N-GMSCs.

9. The preparation method of claim 8, wherein culturing the separated cells comprises providing a solid surface, seeding the cells on the solid surface, culturing the seeded cells, and thereby obtaining a culture comprising cells that are adherent to the solid surface and cells that are not adherent to the solid surface.

10. The preparation method of claim 9, wherein the method further comprises eliminating from the culture the cells that are not adherent to the solid surface.

11. The preparation method of claim 10, wherein the method further comprises dissociating from the solid surface the cells that are adherent to the solid surface by using an enzyme.

12. The preparation method of claim 9, wherein the method further comprises expanding the cultured cells.

## Patentansprüche

1. Verfahren zur Herstellung einer Zusammensetzung, die für eine mesenchymale Stammzell (MSC)-Behandlung eines Menschen geeignet ist, wobei das Herstellungsverfahren umfasst:
a. Trennen eines von einem Menschen erhaltenen Zahnfleischgewebes in Zellen,
b. Sortieren von aus der Neuralleiste stammenden mesenchymalen Gingivastammzellen (N-GMSCs), wobei das Sortieren das Sortieren von Fluoresceinisothiocyanat-positiven Zellen als N-GMSCs umfasst, und
c. Herstellen einer Zusammensetzung, die N-GMSCs umfasst.

2. Herstellungsverfahren nach Anspruch 1, wobei das Trennen des Zahnfleischgewebes in Zellen durch eine Kombination aus einem mechanischen und einem chemischen Verfahren erfolgt, wobei das chemische Verfahren ein enzymatisches Verdauungsverfahren ist.

3. Herstellungsverfahren nach Anspruch 2, wobei das enzymatische Verdauungsverfahren das Verdauen des Zahnfleischgewebes unter Verwendung einer Lösung, die eine Kollagenase und eine Dispase umfasst, und dadurch das Erhalten eines verdauten Zahnfleischgewebes umfasst.

4. Herstellungsverfahren nach Anspruch 3, wobei die Kollagenase Typ I-Kollagenase und die Dispase Dispase II ist.

5. Herstellungsverfahren nach Anspruch 3, wobei das Trennen des Zahnfleischgewebes in Zellen weiter das Herstellen einer Zellsuspension aus dem verdauten Zahnfleischgewebe unter Verwendung eines mechanischen Verfahrens umfasst.

6. Herstellungsverfahren nach Anspruch 5, wobei das mechanische Verfahren das Filtern des verdauten Zahnfleischgewebes umfasst, um die Zellsuspension zu erhalten.

7. Herstellungsverfahren nach Anspruch 6, wobei die Zellsuspension eine Einzelzellsuspension ist.

8. Herstellungsverfahren nach Anspruch 5, wobei das Herstellungsverfahren weiter das Kultivieren der abgetrennten Zellen vor der Sortierung von N-GMSCs umfasst.

9. Herstellungsverfahren nach Anspruch 8, wobei das Kultivieren der abgetrennten Zellen das Bereitstellen einer festen Oberfläche, das Aussäen der Zellen auf der festen Oberfläche, das Kultivieren der ausgesähten Zellen und dadurch das Erhalten einer Kultur umfasst, welche Zellen, die an der festen Oberfläche haften, und Zellen, die nicht an der festen Oberfläche haften, umfasst.

10. Herstellungsverfahren nach Anspruch 9, wobei das Verfahren weiter das Entfernen der Zellen, die nicht an der festen Oberfläche haften, aus der Kultur umfasst.

11. Herstellungsverfahren nach Anspruch 10, wobei das Verfahren weiter das Ablösen der an der festen Oberfläche haftenden Zellen von der festen Oberfläche unter Verwendung eines Enzyms umfasst.

12. Herstellungsverfahren nach Anspruch 9, wobei das Verfahren weiter das Expandieren der kultivierten Zellen umfasst.

## Revendications

1. Procédé de préparation d'une composition appropriée pour un traitement par cellules souches mésenchymateuses (MSC) d'un humain, dans lequel le procédé de préparation comprend :
a. la séparation d'un tissu gingival obtenu à partir d'un humain en cellules,
b. le tri de cellules souches mésenchymateuses gingivales dérivées de la crête neurale (N-GMSC),
dans lequel le tri comprend le tri de cellules positives à l'isothiocyanate de fluorescéine comme étant des N-GMSC, et
c. la préparation d'une composition comprenant des N-GMSC.

2. Procédé de préparation selon la revendication 1, dans lequel la séparation du tissu gingival en cellules est effectuée par une combinaison d'un procédé mécanique et d'un procédé chimique, dans lequel le procédé chimique est un procédé de digestion enzymatique.

3. Procédé de préparation selon la revendication 2, dans lequel le procédé de digestion enzymatique comprend la digestion du tissu gingival en utilisant une solution comprenant une collagénase et une dispase, et ainsi l'obtention d'un tissu gingival digéré.

4. Procédé de préparation selon la revendication 3, dans lequel la collagénase est une collagénase de type I et la dispase est une dispase II.

5. Procédé de préparation selon la revendication 3, dans lequel la séparation du tissu gingival en cellules comprend en outre la préparation d'une suspension cellulaire à partir du tissu gingival digéré en utilisant un procédé mécanique.

6. Procédé de préparation selon la revendication 5, dans lequel le procédé mécanique comprend la filtration du tissu gingival digéré pour obtenir la suspension cellulaire.

7. Procédé de préparation selon la revendication 6, dans lequel la suspension cellulaire est une suspension unicellulaire.

8. Procédé de préparation selon la revendication 5, dans lequel le procédé de préparation comprend en outre la mise en culture des cellules séparées avant le tri de N-GMSC.

9. Procédé de préparation selon la revendication 8, dans lequel la mise en culture des cellules séparées comprend la fourniture d'une surface solide, l'ensemencement des cellules sur la surface solide, la mise en culture des cellules ensemencées, et ainsi l'obtention d'une culture comprenant des cellules qui adhèrent à la surface solide et des cellules qui n'adhèrent pas à la surface solide.

10. Procédé de préparation selon la revendication 9, dans lequel le procédé comprend en outre l'élimination à partir de la culture des cellules qui n'adhèrent pas à la surface solide.

11. Procédé de préparation selon la revendication 10, dans lequel le procédé comprend en outre la dissociation à partir de la surface solide des cellules qui adhèrent à la surface solide en utilisant une enzyme.

12. Procédé de préparation selon la revendication 9, dans lequel le procédé comprend en outre l'expansion des cellules cultivées.
